(19) **Europäisches Patentamt**
**European Patent Office**
**Office européen des brevets**

(11) **EP 4 714 384 A2**

(12) # EUROPEAN PATENT APPLICATION

(43) Date of publication:
25.03.2026 Bulletin 2026/13

(21) Application number: 26155636.9

(22) Date of filing: 23.12.2022

(51) International Patent Classification (IPC):
**A61B 18/22** (2006.01)

(52) Cooperative Patent Classification (CPC):
**A61B 18/22;** A61B 5/0066; A61B 5/0084;
A61B 2017/00061; A61B 2017/00128;
A61B 2018/00642; A61B 2018/00708;
A61B 2018/00785; A61B 2018/00815;
G02B 6/02042; G02B 6/262; G02B 6/3807

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: 30.12.2021 EP 21218295

(62) Document number(s) of the earlier application(s) in
accordance with Art. 76 EPC:
**22844130.9 / 4 456 811**

(71) Applicant: **Optheras A/S**
**3460 Birkerød (DK)**

(72) Inventors:
• **Hermann, Gregers Gautier**
**3460 Birkerød (DK)**

• **Alkeskjold, Thomas**
**3460 Birkerød (DK)**
• **Mogensen, Karin**
**3460 Birkerød (DK)**
• **Petersen, Søren**
**3460 Birkerød (DK)**

(74) Representative: **Guardian
IP Consulting I/S
Diplomvej, Building 381
2800 Kgs. Lyngby (DK)**

Remarks:
This application was filed on 02-02-2026 as a
divisional application to the application mentioned
under INID code 62.

(54) ## DISPOSABLE FIBER-OPTIC APPARATUS

(57) A fiber-optic medical treatment apparatus comprising a medical treatment device and a disposable fiber-optic device, wherein the disposable fiber-optic device comprises an at least double-clad optical fiber having a proximal end and a distal end, the distal end being configured to be advanced through a working channel of an endoscope, wherein the at least double-clad optical fiber has at least one core and at least one cladding; wherein the medical treatment device comprises: a control unit configured to control operation of the medical treatment device; a treatment laser source configured to output treatment laser radiation for treatment of a medical condition; wherein the medical treatment apparatus is configured to output the treatment laser radiation through at least one of the two or more claddings of the at least doubled-clad optical fiber towards tissue to be treated; a sensor module configured to output and receive sensor laser radiation through the at least doubled-clad optical fiber and to optically measure one or more parameters of the treatment based on the received sensor laser radiation; wherein the at least one core of the at least double-clad fiber has a V number of less than 10 at a wavelength of the sensor laser radiation, wherein the medical treatment apparatus is configured to output the sensor laser radiation through the at least one core of the at least doubled-clad optical fiber, and wherein the medical treatment apparatus is configured to time-division multiplex the output of the treatment laser radiation and at least the optical measurement of the one or more parameters.

FIG. 1

## Description

TECHNICAL FIELD

The present disclosure relates to a fiber-optic medical treatment apparatus.

BACKGROUND

Many medical conditions can be treated by applying laser light to tissue to be treated. Often, such treatment is performed by directing treatment laser radiation via an optical fiber towards the tissue to be treated.

In many procedures, an optical fiber is advanced through the working channel of a flexible endoscope. To this end, endoscopes tailored for different types of medical procedures exist. For example, a cystoscope is an endoscope that is configured for performing a cystoscopy where the cystoscope is advanced through the urethra into the urinary bladder. Among other procedures, cystoscopes are used for laser treatment of cancer of the urinary bladder.

Cystoscopes and other types of endoscopes are limited in diameter and, in many procedures, it is desirable that the endoscope is highly bendable. For example, the working channel of cystoscopes typically has a diameter of as little as 1.8 mm. During use, the cystoscope will often be bent to a bending radius of 10 mm radius or to an even smaller bending radius, which may cause the effective diameter of the working channel to be further reduced. Accordingly, it is desirable that the optical fiber used for such procedures is also very thin and highly bendable.

The above challenges are further amplified if, in addition to the treatment laser radiation, additional sensor radiation is to be fed towards the tissue to be treated.

For example, WO 2021/026164 discloses an endoscopic laser energy delivery system, in which different pulse trains of laser radiation at different energy levels are emitted from a distal end of an endoscope and incident on the target. The first laser pulse train is used to form cracks on a surface of a calculi structure, and the second laser pulse train causes fragmentation of the calculi structure after the cracks are formed. The system further comprises a spectroscopy system for analyzing a target composition. A controller of the laser system may automatically program laser therapy with appropriate laser parameter settings based on the target composition.

It remains desirable to feed different types of radiation through the working channel of the endoscope via a fiber-optic device, without unduly degrading the different types of radiation. This may be challenging, as treatment and sensor radiation may impose different and sometimes even opposing requirements or limitations on the fiber-optic device.

Accordingly, it is desirable to provide a fiber-optic treatment apparatus that can be used with an endoscope, such as a cystoscope, having a narrow working channel.

It is further desirable to provide a fiber-optic medical treatment apparatus with an optical fiber that is highly bendable.

As the optical fibers of fiber-optic medical treatment apparatus are typically disposable, i.e. they are often discarded or recycled after a single or at least after few uses, it is further desirable to keep manufacturing costs of the disposable parts of the fiber-optic medical treatment apparatus low.

It is further generally desirable that the physician or other user of the fiber-optic medical treatment apparatus can accurately, reliably and efficiently monitor of one or more aspects of the treatment.

SUMMARY

Aspects of the present disclosure address at least some of the above matters, and others.

In particular, the present disclosure provides embodiments of a fiber-optic medical treatment apparatus comprising a medical treatment device and a disposable fiber-optic device, wherein the disposable fiber-optic device comprises an at least double-clad optical fiber having a proximal end and a distal end, the distal end being configured to be advanced through a working channel of an endoscope, wherein the at least double-clad optical fiber has at least one core and at least two claddings; wherein the medical treatment device comprises:

- a control unit configured to control operation of the medical treatment device;
- a treatment laser source configured to output treatment laser radiation for treatment of a medical condition; wherein the medical treatment apparatus is configured to output the treatment laser radiation through at least one of the two or more claddings of the at least doubled-clad optical fiber towards tissue to be treated;
- a sensor module configured to output and receive sensor laser radiation through the at least doubled-clad optical fiber and to optically measure one or more parameters of the treatment based on the received sensor laser radiation; wherein the at least one core of the at least double-clad fiber has a V number of less than 10 at a wavelength of the sensor laser radiation, wherein the medical treatment apparatus is configured to output the sensor laser radiation through the at least one core of the at least doubled-clad optical fiber, and wherein the medical treatment apparatus is configured to time-division multiplex the output of the treatment laser radiation and at least the optical measurement of the one or more parameters.

Accordingly, the fiber-optic medical treatment apparatus allows different types of radiation to be fed through a single optical fiber, the optical fiber may thus be kept thin and highly bendable. In particular, by time-multiplexing the treatment laser radiation and the optical measurements based on the sensor laser radiation using a single optical fiber, reliable measurements can be obtained during the course of the treatment while avoiding the measurements being corrupted by the treatment laser radiation. In particular, in various embodiments, the medical treatment apparatus is configured to perform treatment as a plurality of successive treatment cycles, each treatment cycle including a treatment period and a sensing period, wherein the medical treatment apparatus is configured to feed the treatment laser radiation through the at least double clad fiber only during the treatment period, and to perform the optical measurements based on the sensor laser radiation only during the sensing period.

By time-division multiplexing the treatment laser radiation and the optical measurements based on the sensor laser radiation using a cladding and a single- or few-mode core of the at least double-clad optical fiber the treatment laser radiation and the sensor laser radiation are separated in time and in space. Accordingly, different requirements imposed by the treatment and sensor radiation can be accommodated by a single optical fiber, e.g. requirements imposed by high-power treatment laser radiation and low-power sensor radiation that is sensitive to signal disturbances, respectively. In particular, this allows optical measurements with a high signal-to-noise ratio to be performed during the treatment, thus allowing a reliable and accurate monitoring of the treatment, resulting in an increased safety and a more gentle treatment with lower risk of over-treating or unintentionally damaging tissue. At the same time the optical fiber may be kept thin and highly bendable and manufacturing costs of the apparatus can be kept relatively low.

Those skilled in the art will recognize still other aspects of the present application upon reading and understanding the attached description.

BRIEF DESCRIPTION OF THE DRAWINGS

The above and other aspects are illustrated by way of example and not limited by the figures of the accompanying drawings, in which like references indicate similar elements and in which:

FIG. 1 diagrammatically illustrates an example of a fiber-optic medical treatment apparatus in accordance with embodiments disclosed herein;
FIG. 2 diagrammatically illustrates an example of a disposable fiber-optic device of a fiber-optic medical treatment apparatus with its optical fiber having been inserted into and advanced through the working channel of an endoscope in accordance with embodiments disclosed herein;
FIG. 3 diagrammatically illustrates an example of a disposable fiber-optic device in accordance with embodiments disclosed herein;
FIG. 4 diagrammatically illustrates an example of an optical combiner module of a connector module of a disposable fiber-optic device in accordance with embodiments disclosed herein;
FIG. 5 diagrammatically illustrates another example of an optical combiner module of a connector module of a disposable fiber-optic device in accordance with embodiments disclosed herein, wherein the at least double-clad optical fiber includes a tapered region;
FIG. 6 diagrammatically illustrates a cross-sectional view of an example of a multi-core double-clad optical fiber for use in a disposable fiber-optic device in accordance with embodiments disclosed herein;
FIG. 7 diagrammatically illustrates operation of the tapered region of the optical combiner module of FIG. 5.
FIGs. 8A-F diagrammatically illustrate cross-sectional views of further examples of an at least double-clad optical fiber for use in a disposable fiber-optic device in accordance with embodiments disclosed herein;
FIGs. 9A-C diagrammatically illustrate longitudinal sectional views of examples of fiber tip designs of an at least double-clad optical fiber for use in a disposable fiber-optic device in accordance with embodiments disclosed herein;
FIG. 10 diagrammatically illustrates a more detailed example of a fiber-optic medical treatment apparatus in accordance with embodiments disclosed herein;
FIG. 11 diagrammatically illustrates an example of an optical combiner module for combining multiple optical paths to be fed through an at least double-clad optical fiber in accordance with embodiments disclosed herein;
FIG. 12 diagrammatically illustrates operation of an example of a fiber-optic medical treatment apparatus in accordance with various embodiments disclosed herein;
FIGs. 13A-B illustrate examples of distance sensing signals obtained by a sensor module of a fiber-optic medical treatment apparatus in accordance with various embodiments disclosed herein.

DETAILED DESCRIPTION

The following describes embodiments of a fiber-optic medical treatment apparatus that mitigate one or more of the above-noted and/or other shortcomings of existing devices or that can at least serve as an alternative to existing devices.

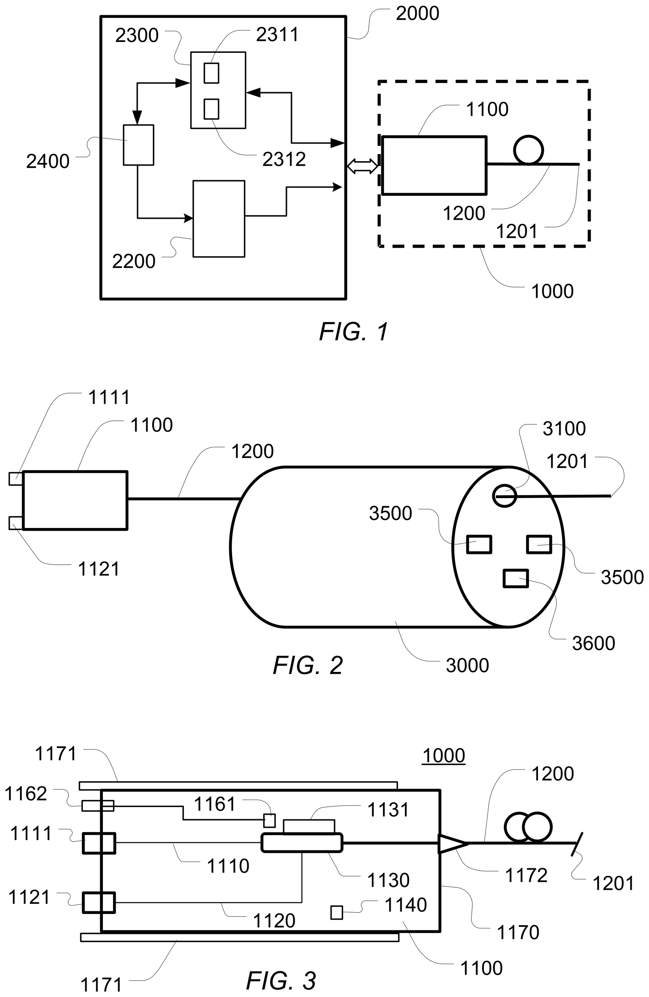

FIG. 1 diagrammatically illustrates an example of a fiber-optic medical treatment apparatus in accordance with embodiments disclosed herein.

The fiber-optic medical treatment apparatus comprises a medical treatment device 2000 and a disposable fiber-optic device 1000. The disposable fiber-optic device 1000 is configured to be detachably and optically coupled to the medical treatment device.

To this end, the disposable fiber-optic device 1000 comprises an at least double-clad optical fiber 1200. The disposable fiber-optic device 1000 may further comprise a connector module 1100 for coupling the at least double-clad optical fiber 1200 to the medical treatment device 2000.

The optical fiber 1200 is at least double-clad, i.e. it may be double-clad or it may be multi-clad with more than two claddings, e.g. triple-clad. Generally, at double-clad optical fiber comprises three layers of optical material. The inner-most layer is called the core. It is surrounded by an inner cladding, which is surrounded by an outer cladding. The three layers are typically made of materials with different refractive indices. The distal end 1201 of the at least double-clad optical fiber 1200 is configured to be advanced through a working channel of an endoscope, such as a cystoscope. Preferably, the core is a single-mode core, single-mode at least at one or more target wavelengths or range of target wavelengths. Optionally, the at least double-clad optical fiber 1200 includes two or more cores, preferably single-mode cores, e.g. a few-mode or single-mode central core and one or more few-mode or single-mode side cores. Optionally, the at least double-clad optical fiber has a medical-grade buffer coating, such as blue EFTE, Teflon, Nylon etc. The optical fiber may have a round inner cladding or a hexagonal or octagonal inner cladding for mode scrambling. The inner cladding may be a multi-core cladding.

The at least double-clad optical fiber 1200 may have a diameter suitable for being inserted into the working channel of an endoscope, i.e. the choice of diameter may depend on the type of endoscope. Suitable diameters may be between 0.1 mm and 1 mm, such as between 0.1 mm and 0.4 mm, preferably between 0.1 mm and 0.3 mm, such as between 0.1 mm and 0.25 mm. Optical fibers having a diameter of 1 mm or less, preferably 0.4 mm or less, such as 0.3 mm or less are sufficiently thin to allow bending with relatively little induced tensile and compressive stress on the fiber edges. The length of the at least double-clad optical fiber may depend on the type of endoscope and/or the type of medical procedure. Suitable lengths may be between 2 m and 4 m.

Generally, at least in some embodiments, the at least double-clad optical fiber which emits the radiation towards the tissue to be treated may have a fiber diameter between 100 $\mu$m and 1000 $\mu$m, such as between 100 $\mu$m and 300 $\mu$m, such as between 150 $\mu$m and 200 $\mu$m. In some embodiments the numerical aperture of the multi-mode cladding, which conveys the treatment laser radiation has a numerical aperture of between 0.17 and 0.50, such as between 0.22 and 0.45. Here, the numerical aperture of the inner cladding may be defined as the square root of the difference between the refractive index squared of the inner cladding and the refractive index squared of the outer cladding, which surrounds the inner cladding.

The distal end 1201 of the at least double-clad optical fiber 1200 may be angled, preferably with rounded edges for easy insertion into the working channel of the endoscope and reduced risk of edges of the at least double-clad optical fiber breaking of during insertion.

The connector module 1100 may be configured for optically connecting the proximal end of the at least double-clad optical fiber 1200 to the medical treatment device 2000. To this end, the connector module 1100 comprises one or more optical connectors. In some embodiments, the connector module 1100 is configured for separately coupling a core and a cladding, respectively, of the at least double-clad optical fiber 1200 to the medical treatment device 2000. To this end, the connector module 1100 may have a first optical connector and a second optical connector. The first optical connector may be configured for detachable connection to a first mating optical connector of the medical treatment device 2000. The second optical connector may be configured for detachable connection to a second mating optical connector of the medical treatment device. An embodiment of the disposable fiber-optic device 1000 will be described in greater detail below with reference to FIGs. 2 and 3.

The medical treatment device 2000 comprises a treatment laser source 2200 configured to output treatment laser radiation for treatment of a medical condition. In particular, the medical treatment apparatus is configured to output the treatment laser radiation through the at least doubled-clad optical fiber 1200 towards tissue to be treated. The treatment laser source 2200 may include a suitable type of laser, e.g. a diode laser, configured to emit treatment laser radiation at a wavelength and power suitable for the medical treatment to be performed. When the medical treatment apparatus is for treatment of tumors of the urinary bladder by ablation and/or photocoagulation, the treatment laser source may comprise a high-brightness diode laser.

The medical treatment apparatus is configured to output the treatment laser radiation via a cladding, in particular the inner cladding, of the at least doubled-clad optical fiber 1200. The cladding may be a multi-mode cladding.

The medical treatment device 2000 further comprises a sensor module 2300. The sensor module is configured to output and receive sensor laser radiation through the at least doubled-clad optical fiber 1200 and to optically measure one or more parameters of the treatment based on the received sensor laser radiation. To this end, the sensor module 2300 may include a sensor laser source 2311 configured to output the sensor laser radiation. The medical apparatus is configured to output the sensor laser radiation through a core of the at least doubled-clad optical fiber 1200. To this end, the at least one core of the at least double-clad optical fiber has a V number of less than 10, preferably less than 8, such as less than 6, such as less than 3 or even 2.4, at a wavelength of the sensor laser radiation core, i.e. the at least one core is a few-mode core or even a single-mode core at a wavelength of the sensor laser radiation. In some embodiments, the sensor laser radiation includes multiple wavelengths. Accordingly, the V number of the least one core of the at least double-clad optical fiber may be different for the different wavelengths of the sensor radiation. For example the core may be a single-mode core at one or some wavelengths of the sensor laser radiation and a few-mode core (or even a multi-mode core) at other wavelengths of the laser radiation. In particular, in some embodiments, the medical treatment apparatus includes more than one sensor module, e.g. an interferometric distance sensor module and a spectroscopic sensor module. The different sensor modules may operate at different wavelengths. In such embodiments, the at least one core of the at least double-clad optical fiber may be a few-mode core with a small V number or even a single-mode core (e.g. having a V number of less than 4, such as less than 3 or even 2.4) at a wavelength of the sensor laser radiation of one of the sensor modules, e.g. of the interferometric sensor module. The at least one core of the at least double-clad optical fiber may be a few-mode core having a larger V number (e.g. between 3 and 10, such as between 3 and 6) at a wavelength of the sensor laser radiation of the other sensor module, e.g. of the spectroscopic sensor module.

The sensor module 2300 may further comprise a detector 2312 for receipt and detection of radiation received from the tissue to be treated, in particular radiation reflected by the tissue in response to being illuminated by the sensor laser radiation from the sensor laser source 2311. The detector 2312 may be configured to receive the radiation from the tissue via the at least double-clad optical fiber 1200 of the disposable fiber-optic device. The detector 2312 may be configured to receive the radiation from the tissue via the at least one core and/or via a cladding of the at least double-clad optical fiber 1200 of the disposable fiber-optic device.

The medical treatment apparatus is configured to time-division multiplex the output of the treatment laser radiation and at least the optical measurement of the one or more parameters. Accordingly, various embodiments of the medical treatment apparatus alternatingly emit the treatment laser radiation and perform optical measurements, i.e. various embodiments of the medical treatment apparatus intermittently interrupt emission - or at least to substantially reduce emission - of the treatment laser radiation to perform the optical measurements, thereby allowing reliable optical measurements to be performed during the treatment. In some embodiments, the medical treatment apparatus is configured to adjust one or more parameters, in particular the output power and/or the duty cycle , of the treatment laser radiation responsive to the measured one or more parameters. Accordingly, the medical treatment apparatus may be configured to control the treatment laser source to resume emission of the intermittently interrupted treatment laser radiation with the adjusted one or more parameters. Generally, it will be appreciated that the time-division multiplexing may comprise a complete interruption of the emission of the treatment laser radiation to perform the optical measurements or at least a substantial reduction of the emitted treatment laser radiation while performing the optical measurements, e.g. a reduction to no more 5% of the output power of the treatment laser radiation used during the treatment cycle, such as a reduction to no more than 1%.

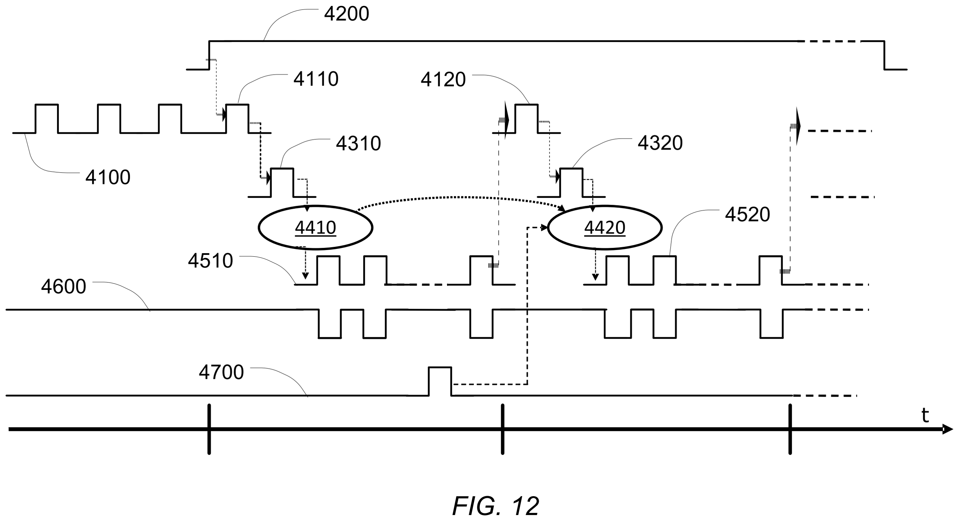

In some embodiments, the medical treatment apparatus is configured to output the treatment laser radiation as pulsed laser radiation, i.e. as a train of treatment laser pulses mutually separated by time intervals between the treatment laser pulses. The medical treatment apparatus is configured to emit the sensor laser radiation during some or all of the time intervals between the treatment laser pulses. An example of the operation of the medical treatment apparatus will be described in more detail below with reference to FIG. 12.

In some embodiments, the sensor laser radiation and the treatment laser radiation have different wavelength. In particular, the sensor laser radiation may have one or more sensor wavelengths in a first wavelength range and the treatment laser radiation may have one or more treatment wavelengths in a second wavelength range, different and, preferably, spaced apart from the first wavelength range. Accordingly, the treatment laser radiation and some or all of the sensor laser radiation may be spatially separated in the core and cladding of the at least double-clad optical fiber, time-division multiplexed as well as spaced apart in wavelength/frequency.

The medical treatment device 2000 comprises a control unit 2400 configured to control operation of the medical treatment device 2000. In particular, the control unit may control operation of the treatment laser source 2200 and of the sensor module. In some embodiments, the control unit controls the time-division multiplexing of the laser radiation and the optical measurements based on the sensor laser radiation. The control unit 2400 may be configured to control various other optical treatment and/or sensor modules of the medical treatment device. The control unit may be configured to control the treatment laser source, sensor laser source and/or other radiation sources and/or sensors responsive to received sensor signals. The control unit may include suitable drivers and/or communications interfaces and a processing unit, e.g. a suitable programmed central processing unit.

In various embodiments, the measured one or more parameters include a distance between the distal end 1201 of the at least double-clad optical fiber 1200 and the tissue to be treated. To this end, in various embodiments, the sensor module 2300 comprises a first optical sensor module, in particular an interferometric distance sensor module, configured to perform interferometric distance sensing of a distance between the distal end 1201 of the at least double-clad optical fiber 1200 and the tissue to be treated. In particular, in some embodiments, the interferometric distance sensor is a common path sensor where the distal end 1201 of the at least double-clad optical fiber is operable as a reflector of a reference optical path, thus providing a particularly compact system, which does not require any additional fibers. The medical treatment device 2000, in particular the control unit 2400 of the medical treatment device, is preferably further configured to control the treatment laser radiation responsive to the measured distance. In particular, the control unit may be configured to control a laser power or another operational parameter of the treatment laser radiation - e.g. a duty cycle of a pulse train of laser cycles, a pulse width or duration of the emission of the laser radiation, or the like - responsive to the sensed distance. For example, the control unit may be configured to control a laser power and/or duty cycle of the treatment laser radiation responsive to the sensed distance, e.g. such that the intensity of the laser radiation impinging on the tissue to be treated remains substantially constant despite the varying distance. In another example, the control unit may be configured to control a laser power or other operational parameter of the treatment laser radiation responsive to the sensed distance such that the energy of laser radiation impinging on the tissue to be treated during a certain time period remains substantially constant despite the varying distance.

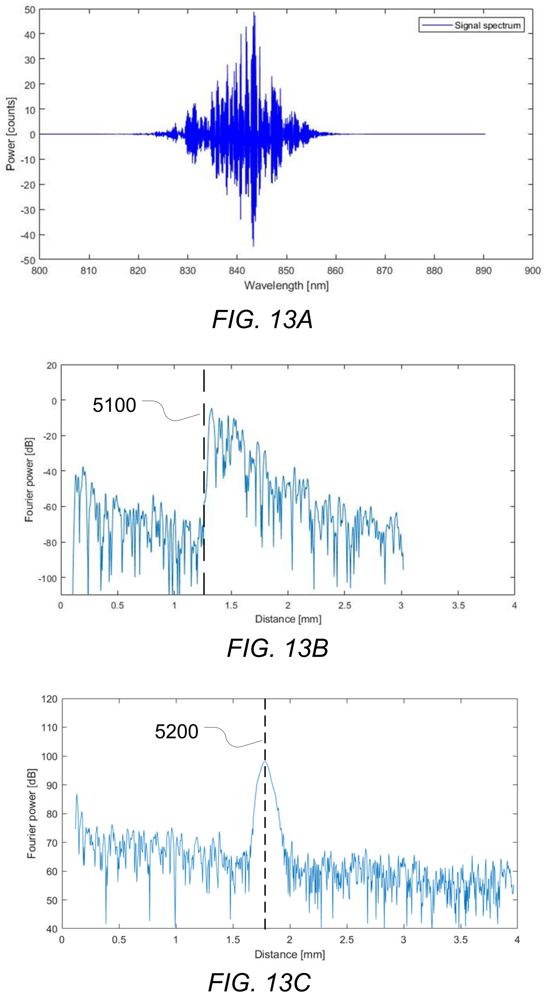

The first optical sensor module may further be configured to detect and distinguish a plurality of different types of material located in front of the distal end of the at least double-clad optical fiber. In such embodiments, the control unit may further be configured to control a laser power and/or other operational parameter of the treatment laser radiation responsive to the detected type of material. In particular, the first optical sensor module may be configured to distinguish between biological tissue, such as soft biological tissue, and non-biological material, in particular surfaces of artificial material, such as an interior or exterior surface of the endoscope. Alternatively or additionally, the first optical sensor module may be configured to detect and distinguish whether the distal end of the at least-double clad optical fiber is immersed in a liquid, in particular water, or whether the distal end is immersed in air. An example of measured signals obtained by an interferometric distance sensor module, and use of the signals for distance sensing and detecting different types of material will be described in more detail below with reference to FIGs. 13A-C.

In some embodiments, the sensor module 2300 comprises a second optical sensor module, in particular a spectroscopic sensor module, configured for spectroscopic analysis of the tissue to be treated and to compute an indicator of a progress of the treatment. To this end, the spectroscopic sensor may be configured for sensing one or more spectral properties of radiation reflected by the tissue to be treated. The medical treatment device is preferably further configured to determine a measure indicative of a status or progress of the treatment from the sensed one or more spectral properties. Preferably, the medical treatment device is configured to control the treatment laser radiation responsive to the determined status or progress, in particular responsive to the sensed distance and the determined status or progress.

The spectroscopic sensor module may comprise a supercontinuum white-light source for illumination through the single-mode core of the at least double-clad optical fiber 1200. Alternatively, the spectroscopic sensor module may comprise a number of WDM multiplexed LEDs for illumination through the single-mode core of the at least double-clad optical fiber 1200. The spectroscopic sensor module may be operable to measure a reflective state of the tissue to be treated, in particular by measuring a wavelength dependent back-reflection, e.g. via the multi-mode inner cladding of the at least double-clad optical fiber 1200. The spectroscopic sensor module may comprise two or more LEDs, that are turned on/off one by one, and the detector may be operable to detect a power of the back-reflected light using a simple photo-detector. The detected power difference between the signals at different wavelengths may be used by the control unit of the medical treatment device to estimate the reflective state of the tissue. The spectroscopic sensor module may measure the Hemoglobin state by measuring the reflected light, for example in the range between 500 nm and 600nm, where Hb absorbs. The spectroscopic sensor module may emit short wavelength light, e.g. blue light, to excite fluorophores in the tissue, and detect the fluorescence. In any event the control unit may use the signals from the spectroscopic sensor to determine optimal settings of the treatment laser.

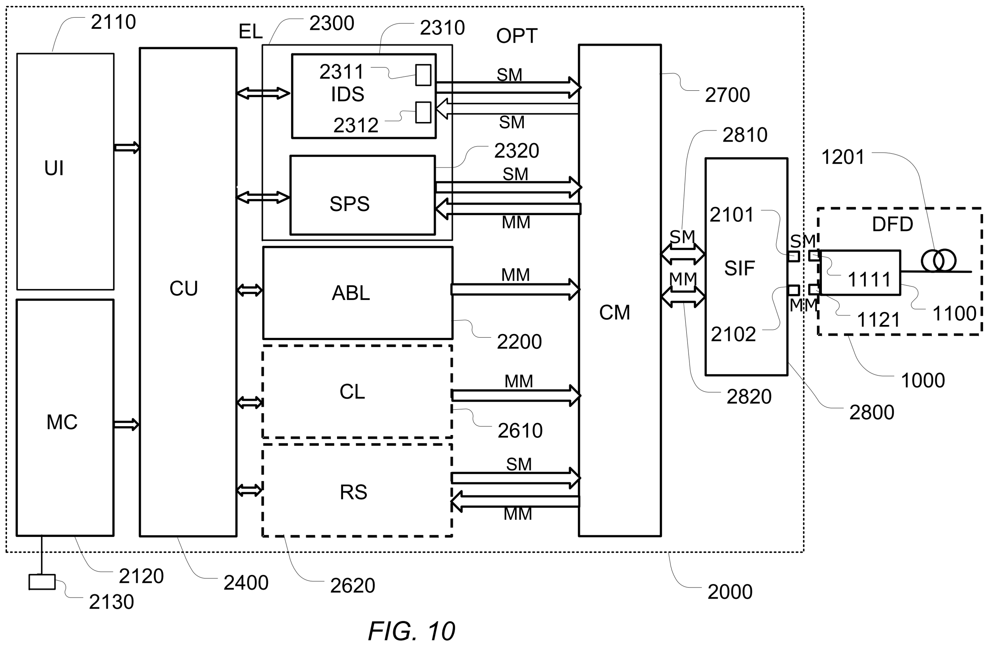

An embodiment of the medical treatment device 2000 will be described in greater detail below with reference to FIG. 10. As will be apparent from the disclosure below, the medical treatment device may include additional or alternative radiation sources and/or additional optical detectors.

In use, the disposable fiber-optic device 1000 is detachably and optically connected to the medical treatment device 2000 via the connector module 1100, and the distal end 1201 of the at least double-clad optical fiber 1200 is inserted into the working channel of an endoscope, e.g. as illustrated in FIG. 2.

FIG. 2 diagrammatically illustrates an example of a disposable fiber-optic device with its at least double-clad optical fiber 1200 having been inserted and advanced into the working channel 3100 of an endoscope 3000. The disposable fiber-optic device comprises an at least double-clad optical fiber 1200 and a connector module 1100, all as described in connection with FIG. 1. In the example of FIG. 2 the distal end 1201 of the at least double-clad optical fiber 1200 extends out of the working channel of the endoscope. In use, the endoscope is inserted into a vessel or organ of a patient, depending on the nature of the medical procedure.

The endoscope 3000 may be inserted with the at least double-clad optical fiber 1200 already inserted in the working channel 3100; alternatively, the at least double-clad optical fiber 1200 may be inserted into the working channel 3100 after insertion of the endoscope 3000 into the target organ or vessel.

In the embodiment of FIG. 2, the connector module 1100 is configured for separately coupling a core and an inner cladding, respectively, of the at least double-clad optical fiber 1200 to the medical treatment device. To this end, the connector module 1100 has a first optical connector 1111 and a second optical connector 1121. The first optical connector 1111 is configured for detachable connection to a first mating optical connector of the medical treatment device and to optically couple the core of the at least double-clad optical fiber to the first mating optical connector. The second optical connector 1121 is configured for detachable connection to a second mating optical connector of the medical treatment device and to optically couple the inner cladding of the at least double-clad optical fiber to the second mating optical connector.

In some embodiments, the treatment laser source of the medical treatment device is optically coupled via the second mating optical connector to the second optical connector 1121 of the disposable fiber-optic device 1000. Accordingly, the second optical connector is preferably configured to receive a treatment laser beam, in particular a treatment laser beam having an output power of between 10 W and 200 W.

Generally, unless specified otherwise, references to numerical values of the power of laser radiation discussed herein, are intended to refer to the peak power of the laser radiation. For example, when the laser radiation is pulsed, the numerical values correspond to the peak power of the individual pulses rather than on the average power of a pulse train. The latter may be smaller than the peak power and related to the peak power by a factor indicative of the duty cycle of the pulse train.

The sensor laser module of the medical treatment device may optically be coupled via a first mating optical connector to the first optical connector 1111 of the disposable fiber-optic device. Accordingly, the first optical connector is preferably configured to receive a sensor laser beam and to output reflected laser radiation.

Endoscopes adapted for various medical procedures are known as such in the art and will not be described in greater detail herein. In particular, different types of endoscopes may have working channels of more or less standardized diameter. Typical working channels of endoscopes have a diameter of between 1 mm and 5 mm. Some endoscopes may further include a camera 3600 and/or one or more illumination lights, e.g. LEDs 3500, arranged at a distal end of the endoscope 3000 so as to allow a physician to observe progress of the medical procedure.

Generally, various embodiments of the disposable fiber-optic device is for treatment of the urinary tract, e.g. for treatment of the urinary bladder, in particular for treatment of bladder cancer. Accordingly, in some embodiments, the endoscope is a cystoscope adapted for insertion into the urinary bladder through the urinary tract. Working channels of cystoscopes typically have a diameter of between 1.5 mm and 2 mm, such as 1.8 mm. A cystoscope typically has to be able to be bent with a small bending radius, e.g. with a bending radius of 10 mm or less, or even 5 mm or less.

FIG. 3 diagrammatically illustrates an example of a disposable fiber-optic device 1000 in accordance with embodiments disclosed herein.

The disposable fiber-optic device 1000 comprises an at least double-clad optical fiber 1200 having a proximal end and a distal end 1201. The distal end 1201 is configured to be advanced through a working channel of an endoscope, e.g. as described in connection with FIG. 2. The at least double-clad optical fiber 1200 has at least one core, an inner cladding surrounding the core, and at least one outer cladding surrounding the inner cladding. In particular, the at least double-clad optical fiber 1200 may be a double-clad optical fiber having one or more cores, an inner cladding and an outer cladding.

The disposable fiber-optic device 1000 further comprises a connector module 1100 for optically connecting the proximal end of the at least double-clad optical fiber 1200 to a medical treatment device (not shown in FIG. 3), e.g. as described in connection with FIGs. 1 and/or 2.

The connector module 1100 comprises a first optical fiber 1110, a second optical fiber 1120, a first optical connector 1111 for detachably and optically connecting the first optical fiber 1110 to medical treatment device, a second optical connector 1121 for detachably and optically connecting the second optical fiber 1120 to the medical treatment device, and an optical combiner module 1300 configured to couple radiation between the first optical fiber 1110 and the at least one core of the at least double-clad optical fiber 1200 and to couple radiation between the second optical fiber 1120 and at least one cladding of the at least double-clad optical fiber 1200.

Generally, detachable connection refers to a connection during normal use performed by a user of the medical treatment apparatus where the connection can be disconnected again during normal use. In particular, detachable connection of an optical connector of the disposable fiber-optic device implies that the corresponding mating connector of the medical treatment device, to which the optical connector is connected, is not destroyed during the connection or disconnection. Preferably, the optical connector of the disposable fiber-optic device is not destroyed during the disconnection either, i.e. the optical connectors of the disposable fiber-optic device can reversibly and repeatedly be connected to the medical treatment device. Optical connection implies that radiation can pass between the medical treatment device and the corresponding optical fiber of the connector module to which the optical connector is coupled.

Generally, in various embodiments, the connector module may comprise a housing 1170. The first optical fiber 1110, the second optical fiber 1120 and the optical combiner module 1300 may completely or partly be accommodated within the housing. The housing 1170 may be a plastic housing or a housing made from another suitable material, e.g. a rigid material or a soft material. The housing may be a box, e.g. made from a rigid material, or it may be provided in another form, e.g. as an elongated flexible sleeve. The housing may form a single compartments or multiple compartments. The housing may have one end configured to be connected to the medical treatment device to thereby connect the first and second optical connectors to corresponding first and second mating optical connectors of the medical treatment device. To this end, the first and/or second optical connector(s), which may be spring-loaded, may be mounted directly on or in a side wall of the housing, thus allowing easy and reliable connection. Alternatively, the first and/or second optical connector(s), which may be spring-loaded, may be mounted to the housing via one or more flexible fiber-optic cables. Accordingly, in general, the components of the connector module may all be accommodated in a single housing or they may distributed between two or more separate housings, which may be interconnected via one or more fiber-optic cables or otherwise.

Generally, the first and/or second optical connector may be spring-loaded. To this end, the first and/or second optical connector may include a spring or other elastic element for holding the first or second optical connector, respectively, in position when connected to the medical treatment device. The spring or other elastic element may be any suitable component for imparting an elastic force, e.g. a flat spring, a coil spring, etc.

The housing 1170 may comprise additional mechanical connectors or guides 1171, e.g. guiderails, that further facilitate a reliable and safe connection, e.g. by ensuring fixation to the medical treatment device with a suitable mechanical force on the spring-loaded connectors. To this end, the guiderails or other mechanical connectors may include a click-on mechanism.

The housing 1170 further includes an opening through which the at least double-clad optical fiber 1200 protrudes out of the housing. The housing 1170 may include a soft cone 1172 or other form of stress and/or bending relief at the opening through which the at least double-clad optical fiber 1200 protrudes out of the housing.

The first optical fiber 1110 may be a single-mode optical fiber or an optical fiber (e.g. a double-clad optical fiber) having a single-mode core or it may include a single-mode optical fiber section. The first optical fiber, or at least a core of the first optical fiber, may be single-mode at least at a predetermined target wavelength, in particular the wavelength used by a sensor module of the medical treatment device, e.g. by an interferometric distance sensor module. The first optical fiber 1110 may be a single fiber section or include multiple fiber sections optically coupled to each other, e.g. spliced or otherwise connected to each other.

The first optical connector 1111 may be a low-reflectivity optical single-mode fiber connector, such as a connector providing physical contact between respective fiber ends of the optical fibers that are optically connected by the optical connector, i.e. the first optical fiber 1110 and a corresponding optical fiber of the medical treatment device. In particular, the first optical connector may be an Angled Physical Contact (APC) Connector. In some embodiments the low-reflectivity optical single-mode fiber connector has a reflectivity of less than -40 dB, such as -50 dB, preferably less than -60 dB, such as less than -70 dB, such as -80 dB. Generally, the first optical connector may be configured to couple few-mode or even single-mode laser radiation, in particular radiation characterizable by a V number smaller than 10, such as smaller than 3, into the first optical fiber. The first optical connector may be configured to couple single-mode laser radiation, having a power of less than 1 W, such as less than 200 mW, such as less than 100 mW, into the first optical fiber.

Accordingly, an efficient optical coupling between single-mode or few-mode fibers with little back-reflection is provided. This is particularly advantageous when the radiation through the core of the at least double-clad optical fiber 1200 is laser radiation for sensor applications, in particular for interferometric measurements such as interferometric distance measurements.

Generally, the terms single-mode radiation or single-mode laser radiation as used herein refer to laser radiation that is transmitted through emitted from a single-mode optical fiber or other single-mode waveguide. Similarly, the terms multi-mode radiation or multi-mode laser radiation as used herein refer to laser radiation that is transmitted through and/or emitted from a multi-mode optical fiber or other multi-mode waveguide.

The second optical fiber 1120 may be a multi-mode optical fiber or include a multi-mode fiber section. The second optical fiber 1120 may be a single fiber section or include multiple fiber sections optically coupled to each other, e.g. spliced or otherwise connected to each other.

The second optical connector 1121 may be a high-power optical multi-mode connector such as an SMA905 connector or a collimated beam connector. A collimated beam connector is a fiber-optic connector including a collimating lens operable to collimate a beam exiting the fiber-optic connector. In particular, the collimating lens may be arranged inside the connector housing of the connector. The collimating lens allows mating of the collimated beam connector with a corresponding collimated beam connector with particularly low power loss. Accordingly, a high-power connection suitable for e.g. high-power treatment laser radiation is provided. The second optical connector may be a free-standing fiber tip connector without physical contact between the fiber ends. Here, the term high-power optical connector is intended to refer to an optical connector adapted for optical connection of optical fibers carrying high-power laser radiation, in particular laser radiation at powers suitable for medical treatment of tissue, e.g. by ablation and/or photocoagulation. The second optical connector 1121 may be adapted for optical connection of optical fibers carrying laser radiation having a power of 10 W or more, such as 100 W or more, such as 200 W or more, such as between 10 W and 500 W. Generally, the second optical connector may be configured to couple multi-mode laser radiation, in particular radiation characterizable by a V number larger than 10, into the second optical fiber.

Generally, two optical connectors may be operable to be connected with each other via a mating sleeve. Accordingly, in some embodiments, each of the mating optical connectors of the medical treatment device that are operable to be coupled to the optical connectors of the disposable fiber-optic device may comprise or be attached to a respective mating sleeve. The mating sleeve of the second mating optical connector of the medical treatment device that is operable to be coupled to the second optical connector of the disposable fiber-optic device may at least in part be made from a suitable material having a sufficiently high melting point and/or low thermal expansion to allow a high-power optical connection. Examples of suitable materials include Tungsten Carbide.

Generally, the first optical connector 1111 and the second optical connector 1121 provide separate optical coupling of the first optical fiber 1110 and the second optical fiber 1120, respectively, with the medical treatment device. In particular, the first optical connector 1111 provides optical coupling of the first optical fiber 1110 with a corresponding first fiber of the medical treatment device, and the second optical connector 1121 provides optical coupling of the second optical fiber 1120 with a corresponding second fiber of the medical treatment device, the corresponding second fiber of the medical treatment device being different from the corresponding first fiber of the medical treatment device.

In some embodiments, the first and second optical connectors are physically separate connectors. In particular, the optical connector 1111 may have a first connector housing, e.g. including a first ferrule, and the second optical connector 1121 may have a second connector housing, e.g. including a second ferrule, different from the first connector housing. In other embodiments, the first optical connector 1111 and the second optical connector 1121 are accommodated in a single connector housing, or they may otherwise be formed as a single combined connector having multiple optical termini. For example, the single connector housing may be a two-ferrule connector housing, such as a Diamond DM4 connector, having multiple optical termini.

The at least double-clad optical fiber 1200 may feed single-mode, low power sensor laser radiation through its core towards the tissue to be treated. The at least double-clad optical fiber 1200 may further feed single-mode reflected radiation through its core from the distal end of the at least double-clad optical fiber via the first optical connector 1111 to the medical treatment device. The reflected radiation may be sensor laser radiation having been reflected by the distal end 1201 of the at least double-clad optical fiber 1200 and/or sensor laser radiation having been reflected by the tissue to be treated and captured by the distal end 1201 of the at least double-clad optical fiber 1200. Accordingly, the core of the at least double-clad optical fiber 1200 is preferably a single-mode core at least at the wavelength or wavelengths of the sensor laser radiation. The at least double-clad optical fiber 1200 may further feed high-power multi-mode treatment laser radiation through its inner cladding towards the tissue to be treated.

For the purpose of the present description, the term "tissue to be treated" is intended to not only refer to the tissue prior to being treated but also to the tissue during the treatment, including to the tissue having already been affected by the treatment. Generally, the tissue to be treated is biological tissue, such as soft tissue.

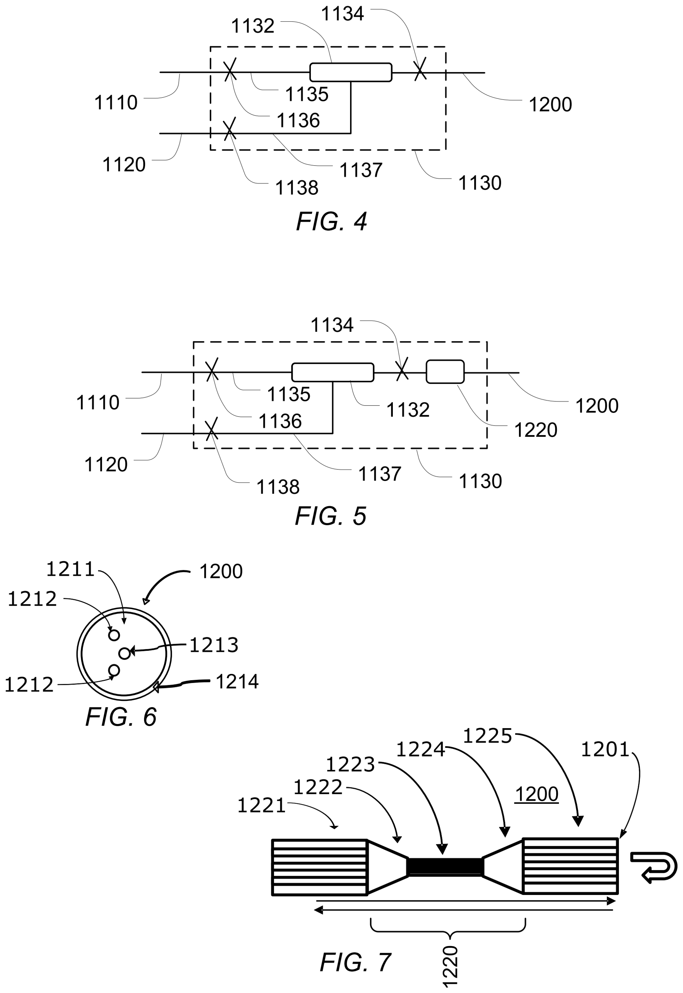

Examples of the optical combiner module 1130 will be described in greater detail with reference to FIGs. 4 and 5, respectively.

Optionally, the connector module 1100 may comprise additional components.

In particular, in some embodiments, the connector module comprises a temperature sensor 1161, e.g. a thermistor, configured to sense the temperature of the optical combiner module 1130. The temperature sensor may be a PTC or NTC sensor. The temperature sensor 1161 may be electrically connectable via electrical connector 1162 to the medical treatment device, thus allowing the medical treatment device to monitor the temperature of the optical combiner module during operation and, optionally, to control operation of the treatment laser source responsive to the sensed temperature. For example, the medical treatment device may be controlled to reduce the power and/or duty cycle of the treatment laser, or even turn the treatment laser off or close a shutter/optical switch, so as to prevent overheating of the optical combiner module 1130.

Alternatively or additionally, the connector module may comprise a cooling member 1131 configured for passive or active cooling of the optical combiner module 1130. The cooling member may e.g. comprise a heat sink, cooling ribs and/or a thermal conductor configured to transport heat into a heat sink of the medical treatment device when the connector module is connected to the medical treatment device.

Yet alternatively or additionally, the disposable fiber-optic device 1000, in particular the connector module 1100, may include an RFID chip 1140 or other form of device-identifier, e.g. a wirelessly readable ID tag, allowing the medical treatment device to automatically register an identifier of the disposable fiber-optic device 1000.

This may be advantageous when the medical treatment device is configured to be operated with different types of disposable fiber-optic devices. It allows the medical treatment device to adapt one or more of its operational parameters to the specific type of disposable fiber-optic device being connected.

FIG. 4 diagrammatically illustrates an example of an optical combiner module 1130 of a connector module of a disposable fiber-optic device in accordance with embodiments disclosed herein.

The optical combiner module 1130 couples radiation between the first optical fiber 1110 of the connector module and a core of the at least double-clad optical fiber 1200 of the disposable fiber-optic device according to an embodiment disclosed herein. The optical combiner module 1130 further couples radiation between the second optical fiber 1120 of the connector module and a cladding, in particular an inner cladding, of the at least double-clad optical fiber 1200.

The first optical fiber 1110 may thus be a single-mode optical fiber, at least at one or more target wavelengths, and the second optical fiber 1120 may be a multi-mode optical fiber.

The optical combiner module 1130 comprises an at least double-clad optical fiber section 1135 having a core and a cladding. The core of the at least double-clad optical fiber section 1135 is a few-mode, preferably a single-mode core at a target wavelength of the radiation received via the first optical fiber. The at least double-clad optical fiber section 1135 may include a low-index acrylate coating, optionally with fluorinated silica (F-SiO2) cladding. The core of the at least double-clad optical fiber section may have a diameter of between 5 $\mu$m and 10 $\mu$m and/or a numerical aperture of between 0.07 and 0.15. The core of the at least double-clad optical fiber section has a V number at a wavelength $\lambda_{sensor}$ of the sensor laser radiation of V < 10 @ $\lambda_{sensor}$, preferably V < 8 @ $\lambda_{sensor}$, such as V < 7 @ $\lambda_{sensor}$, such as V < 2.4 @ $\lambda_{sensor}$.

The V number is a dimensionless parameter which is often used in the context of optical fibers, such as step-index fibers. It is a normalized frequency parameter, which determines the number of modes of an optical fiber, such as a step-index fiber. It is defined as

$$V = \frac{2\pi}{\lambda} a\, NA = \frac{2\pi}{\lambda} a \sqrt{n_{core}^2 - n_{cladding}^2}$$

where $\lambda$ is the vacuum wavelength, a is the radius of the fiber core and NA is the numerical aperture. $n_{core}$ and $n_{cladding}$ refer to the refractive index of the core and of the inner cladding, respectively.

For the purpose of the present description, and unless otherwise stated, the terms single-mode fiber and single-mode core refer to a fiber and core, respectively, that has a V number less than 2.4 at the relevant wavelength. The terms few-mode fiber and few-mode core refer to a fiber and core, respectively, that has a V number larger than 2.4 and less than 10 at the relevant wavelength. The terms multi-mode fiber and multi-mode core refer to a fiber and core, respectively, that has a V number larger than 10.

The optical combiner module 1130 further comprises a first splice point 1136, which couples radiation between the first optical fiber 1110 and the core of the at least double-clad optical fiber section 1135. The first splice point 1136 may be provided with a cladding mode stripper configured for removal of return multi-mode radiation into the first optical fiber 1110. Alternatively or additionally, the first splice point 1136 may be provided with a scattering surface or a high index glue and/or coating.

The optical combiner module 1130 further comprises a multi-mode fiber section 1137 and a second splice point 1138, which couples radiation between the second optical fiber 1120 and the multi-mode fiber section 1137. The multi-mode fiber section 1137 may have a core diameter of between 50 $\mu$m and 100 $\mu$m and/or a numerical aperture of 0.27 or less, e.g. of 0.22 or less. The core of the multi-mode fiber section 1137 may have a V number V > 10.

The optical combiner module 1130 further comprises an optical fiber combiner 1132 configured for coupling radiation between the multi-mode fiber section 1137 and the inner cladding of the at least double-clad optical fiber section 1135. The optical fiber combiner 1132 may be a side combiner, sometimes also referred to as a side pump combiner, i.e. a combiner configured to laterally couple radiation between the multi-mode fiber section 1137 and the inner cladding of the at least double-clad optical fiber section 1135 while the core of the at least double-clad optical fiber section passes through the side combiner. Hence, the side combiner couples/fuses a multi-mode secondary fiber (here the multi-mode fiber section 1137) at an angle to the circumferentially outer side of a pass-through primary fiber (here the at least double-clad optical fiber section 1135). Preferably, the secondary fiber has a diameter smaller than the primary fiber. For example, the double-clad feed through fiber may have a diameter of 200 $\mu$m (or another suitable diameter) and the secondary fiber may have a fiber diameter of 125 $\mu$m (or another suitable diameter smaller than the diameter of the feed-through double-clad fiber). The side coupling may result in an up-conversion of the numerical aperture (NA) of the multi-mode inner cladding of the at least double-clad optical fiber section 1135 and, hence, in the at least double-clad optical fiber 1200, e.g. from NA of 0.22 to about 0.45. This in turn results in a larger spot size of the treatment laser radiation on the tissue to be treated. Moreover, the side combiner allows the sensor laser radiation propagating in the core of the at least double-clad optical fiber section to pass through the side combiner without reflections due to splices or the like.

The optical coupler module 1130 further comprises a third splice point 1134 for coupling the at least double-clad optical fiber section 1135 and the proximal end of the at least double-clad optical fiber 1200, in particular for coupling the cores and claddings of the respective fibers.

FIG. 5 diagrammatically illustrates another example of an optical combiner module 1130 of a connector module of a disposable fiber-optic device in accordance with embodiments disclosed herein. The optical combiner module 1130 is similar to the optical combiner module of FIG. 4 in that it comprises a at least double-clad optical fiber section 1135, a multi-mode fiber section 1137, an optical fiber combiner 1132, a first splice point 1136, a second splice point 1138 and a third splice point 1134, all as described in connection with FIG. 4.

In some embodiments, one or more of the splice points shown in FIGs. 4 and 5 may be omitted. For example, the at least double-clad optical fiber section 1135 may be formed by the proximal end of the at least double-clad optical fiber 1200. Alternatively or additionally, the multi-mode fiber section 1137 may be a portion of the second optical fiber 1120 attached to the second optical connector.

The optical combiner module of FIG. 5 differs from the optical combiner module of FIG. 4 in that it is configured to couple the first and second optical fibers 1110 and 1120, respectively, with a multi-core at least double-clad optical fiber 1200, i.e. the optical combiner module of FIG. 5 is for use with embodiments of a disposable fiber-optic device comprising a multi-core at least double-clad optical fiber.

FIG. 6 diagrammatically illustrates an example of a multi-core double-clad optical fiber 1200. The optical fiber 1200 includes an outer cladding 1214 and an inner cladding 1211, e.g. a multi-mode inner cladding which may be made from quartz. The optical fiber further comprises a central single-mode core 1213 and two single-mode side cores 1212. In other embodiments, the optical fiber may only include a single side core or more than two side cores. Generally, a multi-core optical fiber provides an increased reliability, as it provides multiple redundant paths for the radiation, e.g. sensor laser radiation. This may improve the signal-to-noise ratio of the sensor signals or otherwise improve the reliability of the sensor detection, e.g. allowing continued distance measurements despite parts of the fiber tip being contaminated with tissue or other contaminants.

Again referring to FIG. 5, the optical combiner module 1130 further comprises a tapered fiber region 1220 at the proximal end of the at least double-clad optical fiber 1200. The tapered region 1200 is configured for coupling of radiation between a single core of the at least double-clad optical fiber 1200 and one or more further cores of the at least double-clad optical fiber 1200. The tapered region 1220 may be packaged in a ferrule with air surrounding the taper waist to enable a high numerical aperture so as to support up-converted cladding radiation.

FIG. 7 illustrates operation of the tapered region 1220. In particular, FIG. 7 illustrates the proximal portion 1221 of the at least double-clad optical fiber 1200 extending between the optical fiber combiner 1132 and the tapered fiber region 1220 of the embodiment of FIG. 5, and the distal portion 1225 of the at least double-clad optical fiber 1200 extending out of the housing of the combiner module and being configured for insertion in the working channel of the endoscope. It will be appreciated that FIG. 7 is not drawn to scale and that the distal portion 1225 typically will be much longer than the proximal portion 1221, as the tapered region is preferably arranged with the housing of the connector module.

In the example of FIG. 7, the at least double-clad optical fiber 1200 is a multi-core at least double-clad optical fiber with one central core and two side cores, as illustrated in FIG. 6. However, in other embodiments, the multi-core fiber may have a different number of side cores.

During operation, the central core of the proximal fiber section 1221 carries radiation from the first optical fiber 1110. In particular, the radiation may be a single-mode sensor laser radiation, e.g. for interferometric distance measurement and/or other types of sensor radiation, e.g. for spectroscopy. In the proximal fiber section 1221, the side cores preferably carry no or only minimal radiation.

The optical fiber is tapered down in down-taper region 1222 to a reduced-diameter fiber section 1223 and subsequently again tapered up in up-taper region 1224 to its normal diameter in the distal portion 1225.

The down-tapering and subsequent up-tapering enables coupling of radiation from the central core to the side cores. The length of the reduced diameter section 1223 may be chosen so as to enable equal coupling to all cores or so as to couple all radiation from the central core to the side cores. Accordingly, in the proximal portion 1225, the optical fiber 1200 carries radiation from the first optical fiber 1110 either in the side cores only or in all cores.

At the distal end 1201 of the at least double-clad optical fiber 1200, radiation may partially be reflected backwards by the tip. The remaining radiation exits out of the fiber and is reflected back from the tissue towards which the at least double clad optical fiber 1200 is directed. The reflected light may thus again be captured by the at least double-clad optical fiber 1200 and used for sensor purposes, e.g. for interferometric distance sensing of the distance between the distal end 1201 of the at least double-clad optical fiber and the reflecting tissue and/or for spectroscopic analysis of one or more properties of the reflecting tissue.

Reflected light propagating in the reverse direction through the side cores of the distal portion 1225 will then again be concentrated by the tapered region 1220 into the central core of the proximal portion 1221, thus allowing the reflected radiation to be fed back to a medical treatment device via the first optical fiber and the first optical connector of the connector module.

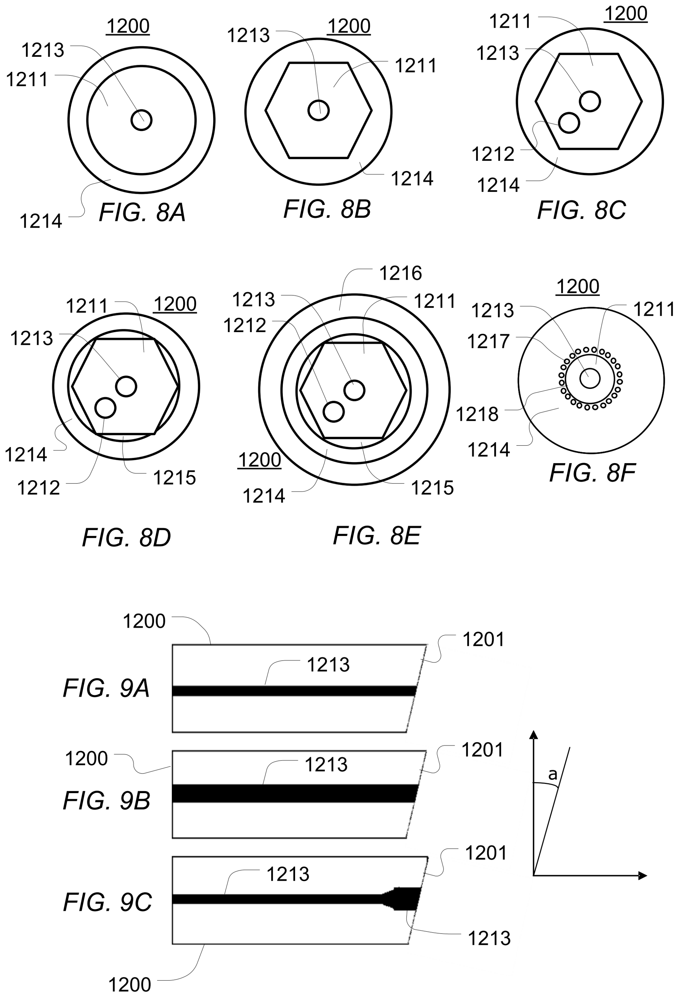

FIGs. 8A-F diagrammatically illustrate further examples of an at least double-clad optical fiber for use in a disposable fiber-optic device in accordance with embodiments disclosed herein.

Generally, in various embodiments, the at least double-clad optical fiber has a core, e.g. a silica core, that is single-mode at least at the wavelength of the sensor laser radiation. The at least double-clad optical fiber has an inner cladding, e.g. a silica cladding, surrounding the core. The inner cladding is operable as a multi-mode core. The at least double-clad fiber further comprises an outer cladding surrounding the inner cladding. The outer cladding may be a silica or polymer cladding, e.g. an FSiO2 layer or a low-index acrylate coating. The at least double-clad optical fiber may further comprise an outermost protective buffer, e.g. a biocompatible coating which may be made from Nylon, Teflon or other suitable material.

In various embodiments, the refractive index $n_c$ of the core is larger than the refractive index of the inner cladding $n_i$, which in turn is larger than the refractive index $n_o$ of the outer cladding.

In particular, FIG. 8A diagrammatically illustrates a cross-sectional view of an example of a single-core double-clad optical fiber 1200. The single-core double-clad optical fiber 1200 includes an outer cladding 1214 and an inner cladding 1211, e.g. a multi-mode inner cladding which may be made from quartz. The optical fiber further comprises a central single-mode core 1213, e.g. from doped-quartz. In some embodiments, the core may be a few-mode core (e.g. characterizable by a V number smaller than 10).

FIG. 8B diagrammatically illustrates a cross-sectional view of another example of a single-core double-clad optical fiber 1200. As in the previous example, the single-core double-clad optical fiber 1200 includes an outer cladding 1214 and an inner cladding 1211, e.g. a multi-mode inner cladding which may be made from quartz. The optical fiber further comprises a central single-mode core 1213. However, in the present example, the inner cladding has a hexagonal cross section. It will be appreciated that other embodiments may have claddings with other cross sectional shapes, e.g. octagonal. A hexagonal or octagonal cladding or another suitably shaped claddings scrambles modes and enables a more uniform intensity distribution in the multi-mode cladding.

FIG. 8C diagrammatically illustrates a cross-sectional view of yet another example of a double-clad optical fiber 1200. The example is similar to the example of FIG. 8B, except that the double-clad optical fiber of FIG. 8C is a two-core double-clad optical fiber having a side core 1212 in addition to the central core 1213. Both cores may be single-mode or few mode cores.

FIG. 8D diagrammatically illustrates a cross-sectional view of yet another example of an at least double-clad optical fiber 1200. The example is similar to the example of FIG. 8C, except that the optical fiber of FIG. 8D further comprises a down-doped quartz layer 1215 surrounding the shaped cladding 1211. It will be appreciated that the single-core example with a shaped cladding of FIG. 8B may also be provided with such a down-doped quartz layer.

FIG. 8E diagrammatically illustrates a cross-sectional view of yet another example of an at least double-clad optical fiber. The example is similar to the example of FIG. 8D, except that the optical fiber of FIG. 8E further comprises an outer coating/buffer 1216, which may e.g. be made of plastic, EFTE, Teflon, Nylon or another suitable material. It will be appreciated that any of the examples of FIGs. 6, 8A - 8D and 8F may also be provided with such an outer buffer/coating.

In some embodiments, the at least double-clad optical fiber is an air-clad optical fiber. In this respect, FIG. 8F diagrammatically illustrates a cross-sectional view of yet another example of an at least double-clad optical fiber 1200, in particular an example of an air-clad optical fiber. The air-clad fiber comprises a core 1213, an inner cladding 1211, an air cladding 1217, and an outer cladding 1214, where the inner and outer claddings are radially separated by the air cladding. The air cladding 1217 is formed by a plurality of air channels that extend along the longitudinal direction of the fiber. The air channels are distributed around the circumference of the inner cladding 1211.

In various embodiments of an air-clad fiber, the refractive index $n_c$ of the core is larger than the refractive index of the inner cladding $n_i$, which in turn is larger than the refractive index $n_{ac}$ of the air cladding. The refractive index $n_o$ of the outer cladding may be substantially equal to the refractive index $n_i$ of the inner cladding.

Air-clad fibers may be provided with a high numerical aperture, even without the need for special plastic coatings, thus allowing use of soft buffer coatings, which reduces micro-bending losses on the fiber. Large numerical apertures, e.g. numerical apertures of up to 0.65@980 nm, may be provided in some embodiments, thus providing a large spot size of the treatment spot. The air-cladding is also operable as a mode scrambler, thereby providing a more uniform beam profile of the beam exiting the fiber at its distal end.

In some embodiments, the air channels of the air cladding may be collapsed in a short portion, e.g. about 50 $\mu$m or less, at the proximal end of the fiber, thereby reducing multi-mode splicing losses.

In some embodiments, the air channels of the air cladding may be collapsed in a portion, e.g. about 50 $\mu$m or more, at the distal end of the fiber, thereby allowing beam expansion of the multi-mode beam before exiting the fiber.

In some embodiments, the air-clad fiber comprises a fluorine-doped layer 1218 radially inward of the air-cladding, thereby facilitating capturing of more light at the splice points, reducing splice losses and reducing heating issues at the splice points. In some embodiments, the air-clad fiber with fluorine-cladding may have a numerical aperture of between 0.22 and 0.30, matching or exceeding the numerical aperture of the side combiner of the connector module.

Generally, in various embodiments, e.g. in the embodiments of FIGs. 6 and FIGs. 8A-F, the core or cores of the at least double-clad optical fiber may have different dimensions, e.g. as illustrated in FIGs. 9A-C. The choice of core dimensions may strike a balance between bendability of the fiber, signal-to-noise ratio, etc. A large core diameter of e.g. more than 5 $\mu$m, such as between 10 $\mu$m and 15 $\mu$m provides a high signal strength for e.g. an interferometric distance sensor signal. A smaller core diameter of less than 10 $\mu$m, e.g. of about 5 $\mu$m, facilitates tighter bending. In some embodiments, the fiber may have a core diameter of between 5 $\mu$m and 10 $\mu$m with a depressed cladding for improved bending.

FIGs. 9A-C diagrammatically illustrate examples of fiber tip designs of an at least double-clad optical fiber for use in a disposable fiber-optic device in accordance with embodiments disclosed herein. Each of FIGs. 9A-C shows a distal portion of the at least double-clad optical fiber 1200 including the distal end 1201. In each of the examples, the distal end is provided as an angled tip, preferably with rounded edges. The tip is angled at an angle a which may e.g. be chosen between 3° and 10°.

Generally, in various embodiments, the optical fiber has a diameter small enough to be advanced through the working channel of an endoscope, such as a cystoscope. In some embodiments, the optical fiber 1200 has a diameter of 2 mm or less, such as 1.5 mm or less, such as 1 mm or less, such as 0.75 mm or less, such as 0.6 mm or less.

For small fibers, it is often not feasible to attach any lenses or other optical elements to the distal end of the fiber, i.e. the optical system is preferably designed to accommodate a situation where the radiation diverges out of the fiber tip towards the tissue to be treated.

FIG. 9A illustrates a single-core fiber having a single-mode core with numerical aperture of 0.14 and a diameter of 6 $\mu$m, thus allowing tight bending.

FIG. 9B illustrates a single-core fiber having a single-mode core with numerical aperture of 0.075 and a diameter of 10 $\mu$m, thus providing a high signal strength for e.g. an interferometric distance sensor signal. Generally, in some embodiments, the at least double-clad optical fiber may have a large core (e.g. with a diameter between 10 $\mu$m and 15 $\mu$m) step index fiber with a surrounding low-index cladding trench, to allow tighter bending.

FIG. 9C illustrates a single-core fiber having a single-mode core with an expanded tip, such as a thermally expanded tip, e.g. expanded from a core diameter of between 5 $\mu$m and 8 $\mu$m, such as 6 $\mu$m, to an expanded core diameter of e.g. 10 $\mu$m. Such a tip design provides a reduced core numerical aperture through a thermal process while causing the fiber mode to expand at the fiber end.

FIG. 10 diagrammatically illustrates a more detailed example of a fiber-optic medical treatment apparatus in accordance with embodiments disclosed herein.

The fiber-optic medical treatment apparatus comprises a medical treatment device 2000 and a disposable fiber-optic device 1000, all as described in connection with FIG. 1.

Embodiments of a disposable fiber-optic device are described in connection with FIGs. 2 - 5. The disposable fiber-optic device 1000 is configured to be detachably and optically coupled to the medical treatment device 2000. The disposable fiber-optic device 1000 comprises an at least double-clad optical fiber 1200 and a connector module 1100, all as described in connection with any of the previous figures.

The example fiber-optic medical treatment apparatus is configured for treatment of cancer of the urinary bladder by laser ablation and/or photocoagulation using a cystoscope. It will be appreciated, however, that other embodiments of the fiber-optic medical treatment apparatus may be configured for other types of medical procedures and/or for use with other types of endoscopes.

The medical treatment device 2000 comprises various laser sources, optical detectors and accompanying control circuitry, user interfaces, etc. The medical treatment device 2000 may be embodied with all its modules accommodated in a single housing or with respective modules accommodated in different housings.

The medical treatment device 2000 comprises a user-interface module 2110, e.g. comprising a display, such as a touch-sensitive display and suitable user input devices, such as a keyboard, touch screen etc., allowing an operator to control user-controllable functions of the medical treatment device and to allow the medical treatment device to output information relevant for the treatment. In some embodiments, the user-interface module comprises an audible and/or visible power/progress indicator configured to output an audible and/or visual indication indicative of a current laser power of the treatment laser radiation or a current status of the treatment. For example the power indicator may indicate the output power of the treatment laser or the measured treatment status, such that the physician knows when to move the fiber to the next spot. An audible indicator may indicate the power level or treatment status by the volume or pitch of the audible signal, by a repetition rate of repeated tones, or in another suitable manner.

The medical treatment device 2000 may further comprise one or more control devices 2120, such as a foot pedal, e.g. to allow a physician to activate a treatment laser while manipulating the cystoscope.

The medical treatment device further comprises a control unit 2400 electrically and/or otherwise communicatively connected to the user-interface module 2110 and to the one or more control devices 2120. The control unit 2400 comprises circuitry configured to control various optical treatment and/or sensor modules of the medical treatment device. In particular, the control unit may be configured to control a treatment laser source, a sensor laser source and/or other radiation sources and/or sensors responsive to input received from the user-interface module and/or the one or more control devices. The control unit may further control the treatment laser source, sensor laser source and/or other radiation sources and/or sensors responsive to received sensor signals. The control unit may include suitable drivers and/or communications interfaces and a processing unit, e.g. a suitable programmed central processing unit.

The medical treatment device 2000 comprises an treatment laser source 2200 configured to output treatment laser radiation suitable for treating tumor tissue of the urinary bladder by ablation and/or photocoagulation. In some embodiments, the treatment laser source is configured to output treatment laser radiation at a wavelength which is preferably chosen to provide high water and Hemoglobin absorption and/or low water and high Hemoglobin absorption, such as at about 980 nm or at another suitable wavelength, e.g. in the range between 750 nm and 1000 nm, such as between 790 nm and 820 nm or between 900 nm and 1000 nm, such as between 900 nm and 940 nm, e.g. between 905 nm and 925 nm, or between 970 nm and 1000 nm, such as between 970 nm and 990 nm. The treatment laser radiation may have a suitable power such as between 10 W and 200 W. Treatment laser radiation from the treatment laser source 2200 is preferably fed through the second optical connector 1121 and the cladding of the at least double-clad optical fiber 1200 of the disposable fiber-optic device 1000.

The medical treatment device further comprises a sensor module 2300. The sensor module comprises a first optical sensor module 2310 configured for interferometric distance sensing of the distance between the distal end of the at least double-clad optical fiber 1200 and the tissue to be treated.

Preferably, the medical treatment device 2000 may be configured to control the treatment laser source 2200 responsive to the sensed distance between the distal end of the at least double-clad optical fiber 1200 and the tissue to be treated, e.g. by reducing the output power and/or duty cycle of the treatment laser radiation or even shutting down the treatment laser radiation when the fiber tip gets close to the tissue. This may prevent unintended damage to the tissue to be treated.

Interferometric distance sensing may be performed using broadband interferometry known as such from optical coherence tomography (OCT). To this end, the first optical sensor module may comprise a suitable sensor laser source 2311 configured to output single-mode laser radiation in a suitable wavelength, e.g. selected in the range between 800 nm and 1100 nm. In applications such as cystoscopy, it is preferred that the wavelength of the sensor laser radiation is chosen at low-water absorption wavelengths, such as wavelengths smaller than 900 nm or wavelength at about 1050 nm.

The sensor laser radiation may have an output power of between 1 mW to 500 mW, i.e. considerably smaller, e.g. by at least 2 or 3 orders of magnitude, than the output power of the treatment laser radiation.

The first optical sensor module 2310 further comprise a spectrometer 2312 to perform broadband interferometry. To this end, the first optical sensor module receives reflected radiation back via the disposable fiber-optic device, in particular reference radiation reflected by the tip of the at least double-clad optical fiber 1200 and radiation reflected by the tissue to be treated and captured by the tip of the at least double-clad optical fiber 1200. Based on the reflected radiation, the medical treatment device determines the distance between the distal end of the at least double-clad optical fiber 1200 and the tissue to be treated in a manner known as such in the art. Accordingly, the first optical sensor module 2310 may output sensor laser radiation via the single-mode or few mode core of the at least double-clad optical fiber and receive reflected radiation via the single-mode or few mode core of the at least double-clad optical fiber as a basis for the distance measurement.

Various embodiments of the interferometric distance sensor of the first optical sensor module 2310 provide an axial resolution of between 5 $\mu$m and 200 $\mu$m, such as between 10 $\mu$m and 150 $\mu$m, e.g. between 50 $\mu$m and 100 $\mu$m.

The interferometric distance sensor of the first optical sensor module 2310 may be implemented in different ways. In some embodiments the interferometric distance sensor employs spectral domain OCT using a SLED and a spectrometer. The inventors have found that such a system provides sufficient axial resolution with a bandwidth of the SLED of about 10 nm - 30 nm. Accordingly, the spectrometer only needs to cover a relatively small bandwidth as well. Preferably, the spectrometer has a resolution of between 0.01 nm - 0.05 nm, thereby obtaining a sufficient axial range suitable for distance measurements during treatment. The interferometric distance sensor may operate at wavelengths in the range between about 800 nm and about 900nm where CMOS detectors/spectrometers have good sensitivity and In-GaAs or GaAs SLEDs are readily available. This maximum axial range of the distance sensor may be between about 3 mm to 10 mm, such as between about 3 mm to 9 mm.

In an alternative embodiments, the interferometric distance sensor may employ spectral domain OCT using a swept-source laser and a detector. This embodiment has the advantage of an extended maximum axial range to between about 10 mm and 20 mm. Output powers of 100 mW and higher can be obtained using suitable laser sources, e.g. a 1060 nm InGaAs or GaAs swept source laser of 1-2 mW, and by seeding the laser radiation to a Yb-fiber amplifier or amplifier chain so as to boost the output power to between 2 mW and 500 mW. This embodiment may be implemented similar to conventional swept-source OCT systems, with synchronized data acquisition to the laser sweep. The sweep range may be selected to be between 10 nm and 40 nm, such as about 20 nm or less, and the sweep frequency may be selected to be between 1 kHz and 100 kHz, such as 10 kHz or less.

In order to accommodate measurements via a fiber advanced through a flexible endoscope, which is bent and moved around during treatment, various embodiments of the interferometric distance sensor employ a common path interferometric measurement, where the at least double-clad optical fiber 1200 is used both a reference path and as a sample path. Reflection from the distal end of the at least double clad optical fiber is used as reference signal. Hence, a separate reference arm is avoided, and thereby reducing system complexity and costs.

In such embodiments, a suitable amplitude of the reference signal ensures a good interference signal on the spectrometer. To this end, a suitable reference signal amplitude may be obtained by selecting a suitable fiber tip angle of the distal end of the at least double-clad optical fiber, e.g. an angle between 2 and 10 degrees, depending on the fiber type and laser power.

In various embodiments, the radiation from the sensor laser source 2311 of the interferometric distance sensor 2310 as well as the back-reflected radiation are fed through the single-mode core of the at least double-clad optical fiber 1200 and through the first optical connector 1111 of the disposable fiber-optic device 1000. The inventors have realized that the signal path from the spectrometer of the interferometric distance sensor to the distal end of the at least double-clad optical fiber has low back-reflection losses, preferably less than -60dB. Accordingly, in some embodiments, the first optical connector 1111 of the disposable fiber-optic device, through which the sensor signals for interferometric distance measurement are fed, is preferably a low-reflection connector, e.g. a fiber optical connector with physical contact between fibers, optionally an angled connector such as an E2000 connector.

The sensor module 2300 may preferably comprise a second optical sensor module 2320 configured for spectroscopic analysis of the tissue to be treated. In particular, spectral properties of radiation reflected by the tissue to be treated may be used as an indicator of the progress of the treatment. To this end, the medical treatment device may be configured to measure tissue state using reflectance spectroscopy, e.g. using white-light reflectance spectroscopy.

As the tissue is photo-treated, it changes state from an absorbent state to a more scattering state. Low wavelength light will be more reflected than long wavelength light, and by measuring the difference in reflected power at one or more wavelengths, the scattering state of the tissue can be estimated. Measuring the difference in power between two or more wavelengths of back-reflected radiation thus yields a measure of the photocoagulation state of the tissue and, hence, of the status or progress of the treatment. Accordingly, in various embodiments, the medical treatment device is configured to monitor the status of coagulation caused by the treatment laser using reflectance spectroscopy.

In some embodiments, the second sensor module 2320 comprises an illumination source using a supercontinuum laser, e.g. covering from a wavelength range between 400 nm and 1100 nm, such as between 400 nm and 800 nm. Alternatively the illumination source may us use one or more single-colored LEDs at respective wavelengths, e.g. at one or more of the following wavelengths: about 400 nm, about 500 nm, about 600 nm and about 700 nm and/or other wavelengths in the range between 400 nm and 700 nm.

In any event, the second optical sensor module 2320 is configured to receive radiation reflected by the tissue to be treated. The second optical sensor module 2320 may thus comprise a suitable detector for measuring the reflected radiation. The detector may e.g. comprise a grating CCD/CMOS spectrometer, e.g. with up to 1 nm resolution, covering a suitable wavelength range, e.g. between 400 nm and 1100 nm, such as between 400 nm and 800 nm. Alternatively, the detector may include a photodetector, which may be time-multiplexed with a plurality of LEDs.

To maintain good signal strength of the radiation reflected back from the tissue, it is preferred that the illumination light passes through the first optical connector 1111 and through the single-mode core of the at least double-clad optical fiber 1200 of the disposable fiber-optic device 1000, i.e. that the illumination light shares the same path through the disposable fiber-optic device as the interferometric distance measurement radiation. Spectroscopy is a non-interferometric method, and hence more limited by noise than an interferometric distance measurement. Therefore, in various embodiments, the multi-mode cladding of the at least double-clad optical fiber 1200 of the disposable fiber-optic device 1000 captures the reflected light of the spectroscopic illumination, i.e. the second optical sensor module 2320 receives radiation reflected back from the tissue to be treated responsive to being illuminated by the illumination source of the second optical sensor module 2320 via the cladding of the at least double-clad optical fiber 1200 and via the second optical connector 1121 of the disposable fiber-optic device 1000.

Accordingly, the medical treatment apparatus may display or otherwise output a progress indication or even automatically control the treatment laser source 2200, e.g. to prevent unintended overtreatment which might otherwise result in undesired tissue damage. In some embodiments, the second optical sensor module 2320 may output sensor laser radiation via the single-mode or few mode core of the at least double-clad optical fiber and receive reflected sensor laser radiation via the single-mode or few mode core or via the multi-mode cladding of the at least double-clad optical fiber. The wavelengths used for spectral analysis may preferably be chosen at low-water absorption wavelengths, such as wavelengths smaller than 900 nm or wavelengths at about 1050 nm. In particular, in some embodiments, the wavelengths used for spectral analysis may preferably be chosen such that they correspond with absorption maxima hemoglobin and/or another suitable molecular marker.

In some embodiments, the medical treatment device may include additional or alternative optical components, e.g. one or more of the following further components: Some embodiments of the medical treatment device 2000 comprise at least one additional treatment laser 2610, e.g. configured for cutting tissue rather than for ablation and/or photocoagulation. The cutting laser may output treatment laser radiation at a suitable wavelength, e.g. infrared radiation, such as at 1940 nm, which is absorbed by water. Treatment laser radiation from the cutting laser 2610 is preferably fed through the second optical connector 1121 and the cladding of the at least double-clad optical fiber 1200 of the disposable fiber-optic device 1000. In some embodiments, the additional treatment laser 2610 outputs treatment laser radiation configured to be absorbed by water in the vicinity of the tissue to be treated and to facilitate removal of treated tissue from the treatment site. To this end, the additional treatment laser 2610 may be configured to output treatment laser radiation at a wavelength between 1000 nm and 2000 nm, such as between 1200 nm and 2000 nm.

Alternatively or additionally, the medical treatment device 2000 may comprise a Raman sensor 2620 or other suitable sensor configured for determining properties of a tumor to be treated. The Raman sensor may e.g. employ a 785 nm 300 mW Raman laser. Sensor laser radiation from the Raman laser is preferably fed through the first optical connector 1111 and the single-mode core of the at least double-clad optical fiber 1200 of the disposable fiber-optic device 1000. Return radiation to the Raman sensor is preferably fed through the cladding of the at least double-clad optical fiber 1200 and the second optical connector 1121 of the disposable fiber-optic device 1000.

Yet alternatively or additionally, the medical treatment device may include a pilot light source, e.g. a pilot laser source or other type of light source adapted to emit visible light suitable as pilot beam to illuminate the portion of the tissue to be treated and to serve as aiming guide to the physician or other user of the apparatus. The pilot beam may be fed through the at least double-clad optical fiber 1200, e.g. through the core and/or the inner cladding of the at least double-clad optical fiber 1200. In some embodiments, the pilot beam may be multi-colored. In particular, the pilot beam may include two or more color components detectable by a camera of the medical treatment apparatus, e.g. including at least two detectable color components that are at least 40 nm apart. The at least two color components may be part of a broad-band emission spectrum having a bandwidth of 40 nm or more, where the intensity of the emitted pilot light may be substantially uniform across the bandwidth or vary across the bandwidth. The two detectable color components may each have an intensity high enough for them to be detectable by a camera or similar detector of the medical treatment apparatus such that the spot illuminated by the pilot beam is visible to the human observer in a camera image as an illuminated spot having a color resulting as a mix of said at least two color components, e.g. as a white or substantially white spot.

In some embodiments, the apparatus is configured to control a visible attribute, in particular an intensity, a color and/or color distribution, of the visible pilot light responsive to one or more of: a user command, an operational parameter of the first treatment laser radiation and a sensor signal obtained by a sensor module. For example, the apparatus may control the visible attribute of the pilot beam, e.g. change a color and/or intensity of the pilot beam, responsive to the user operating a foot pedal or other input device for activating the treatment laser radiation. Alternatively or additionally, the apparatus may control the visible attribute responsive to one or more operational parameter of at least the first setting, e.g. a selected laser power and/or duty cycle and/or whether the apparatus is currently emitting the first or second treatment laser radiation. Yet alternatively or additionally, the apparatus may control the visible attribute responsive to one or more sensor signals, e.g. responsive to a sensed distance between the distal end and the tissue to be treated, a detected tissue property. The detected tissue property may e.g. be indicative of the presence of tissue within a range of the distal end and/or a sensed stage of photocoagulation or ablation and/or the like. The tissue property may be sensed by sensing one or more spectral properties of radiation reflected by the tissue to be treated or otherwise. Accordingly, the intensity, color or other visible property of the pilot beam may be used to indicate one or more conditions or operational parameters to the user.

In some embodiments, the medical treatment apparatus comprises a handheld communication device 2130. The handheld communication device may be communicatively coupled to the medical treatment device 2000, e.g. via a wired or wireless connection. The handheld communication device comprises a user input device, e.g. a push button, that is configured to receive user input from a patient during treatment with the medical treatment apparatus. The user input may be indicative of pain or discomfort experienced by the patient during the treatment. To this end the patient may be instructed to press a button if the patient experiences pain or discomfort during the treatment. In some embodiments, the user input device may be configured to measure a force or pressure applied to the user input. The medical treatment device may thus derive a degree of pain or discomfort from the measured force or pressure. The control unit 2400 of the medical treatment device may thus be configured to select a laser power or a pulse duration or duty cycle or another operational parameter of the treatment laser radiation at least in part responsive to the received user input. To this end, the control unit may compare the information from the user device to a current, calculated or selected operational parameter of the treatment laser radiation and adjust the operational parameter to minimize pain of the patient. For example, the control unit may compare the information from the user device to the current, calculated or selected laser power and adjust a maximum power of the treatment laser radiation to minimize pain of patient.

The medical treatment device 2000 further comprises an optical combiner module 2700 having optical interfaces to the treatment laser source 2200, the first optical sensor module 2310 with an interferometric distance sensor, and the optional second optical sensor 2320. In embodiments with additional optical treatment and/or sensor units, the optical combiner module also has optical interfaces to any such units, e.g. to a cutting laser 2610 and/or a Raman sensor 2620.

As was described above, the treatment laser source 2200 emits relatively high-power treatment laser radiation to be fed through the multi-mode cladding of the at least double-cad optical fiber while the interferometric distance sensor of the first optical sensor module 2310 emits and receives low-power radiation via the single-mode or few-mode core of the at least double-cad optical fiber. The second optical sensor module 2320 emits radiation to be fed through the single-mode or few.-mode core of the at least double-clad optical fiber 1200 of the disposable fiber-optic device, and receives radiation through the cladding of the at least double-clad optical fiber 1200 of the disposable fiber-optic device.

Accordingly, the optical combiner module 2700 is configured to combine the optical paths fed through the core of the at least double-clad optical fiber 1200 via the first optical connector 1111 of the disposable fiber-optic device 1000 with one another. The optical combiner module 2700 is further configured to combine the optical paths fed through the cladding of the at least double-clad optical fiber 1200 via the second optical connector 1121 of the disposable fiber-optic device 1000. To this end, the optical combiner module 2700 may comprise suitable fused fiber combiners for coupling the signal paths fed through the single-mode core of the at least double-clad optical fiber 1200. Similarly, the optical combiner module may comprise suitable free-space components, lenses and dichroic filters to perform multi-mode power and wavelength splitting/combination of the radiation fed through the cladding of the at least double-clad optical fiber 1200.

The optical combiner module 2700 comprises two optical interfaces to be coupled to the disposable fiber-optic device 1000: a single-mode or few-mode interface 2810 (e.g. characterizable by a V number smaller than 10) and a multi-mode interface 2820 (e.g. characterizable by a V number larger than 20), respectively.

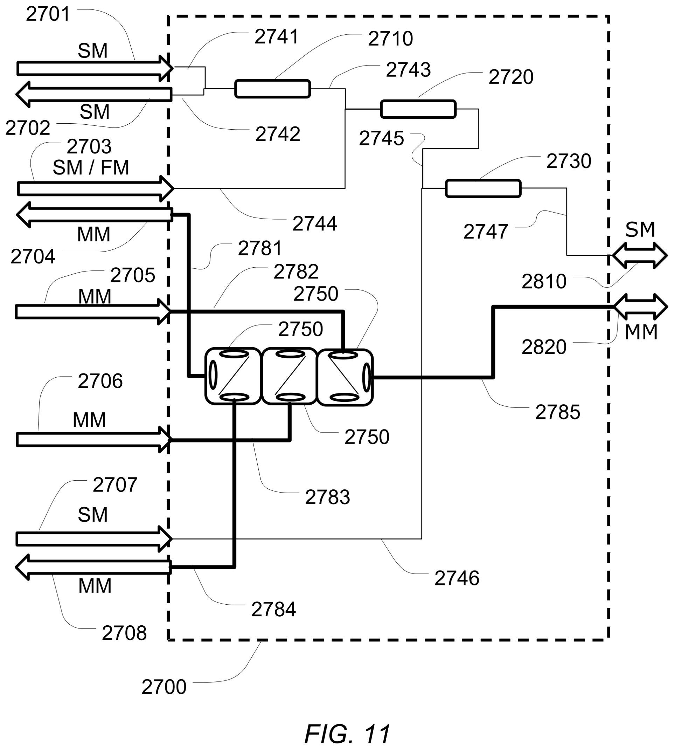

An example of an optical combiner module will be described in more detail below with reference to FIG. 11.

The medical treatment apparatus preferably comprises a sacrificial interface module 2800 having corresponding optical interfaces to interface with the single-mode or few-mode interface 2810 and the multi-mode interface 2820, respectively. The sacrificial interface module 2800 optically connects the single-mode or few-mode interface 2810 with a first mating optical connector 2101 to which the first optical connector 1111 of the disposable fiber-optic device 1000 is connectable. Moreover, the sacrificial interface module 2800 optically connects the multi-mode interface 2820 with a second mating optical connector 2102 to which the second optical connector 1121 of the disposable fiber-optic device 1000 is connectable. During operation of the medical treatment apparatus, different disposable fiber-optic devices may interchangeably be connected to the mating first and second optical connectors 2101 and 2102, respectively, of the sacrificial interface module 2800, as the disposable fiber-optic device 1000 is typically a single-use device that is replaced by a new disposable fiber-optic device between each treatment. Therefore, the first and second mating optical connectors of the sacrificial interface may be subject to wear. Accordingly, provision of a sacrificial interface module 2800 that can relatively easily be replaced facilitates maintenance of the medical treatment device 2000.

It will be appreciated that the radiation form/to the respective optical modules of the medical treatment device may not necessarily be fed through the disposable fiber-optic device concurrently. Instead, at least some of the radiation is time-multiplexed in a suitable manner to avoid that they interact with each other in an undesired manner. An example of time-division multiplexed operation will be described in more detail below with reference to FIG. 12.

FIG. 11 diagrammatically illustrates an example of an optical combiner module for combining multiple optical paths to be fed through an at least double-clad optical fiber. The optical combiner module 2700 has optical interfaces 2701-2708 to the various optical components of the medical treatment device, in particular to the treatment laser source, and to the sensor module.

In the illustrated embodiment, the optical combiner module 2700 has an optical interface 2705 for receipt of multi-mode treatment laser radiation from the treatment laser source of the medical treatment device. The optical combiner module 2700 further has an optical interface 2701 for receipt of single-mode sensor laser radiation from the interferometric distance sensor module of the medical treatment device and an optical interface 2702 for feeding single-mode response radiation, which has been reflected by the tissue to be treated in response to being illuminated by the sensor laser radiation from the interferometric distance sensor module, back to the interferometric distance sensor module of the medical treatment device. Similarly, in embodiments, where the medical treatment device further comprises a spectroscopic sensor module, the optical combiner module 2700 further has an optical interface 2703 for receipt of single-mode or few-mode sensor laser radiation from the spectroscopic sensor module of the medical treatment device and an optical interface 2704 for feeding multi-mode response radiation, which has been reflected by the tissue to be treated in response to being illuminated by the sensor laser radiation from the spectroscopic sensor module, back to the spectroscopic sensor module of the medical treatment device.

Moreover, in embodiments, where the medical treatment device further comprises a further treatment laser source, e.g. a cutting laser, the optical combiner module 2700 further has an optical interface 2706 for receipt of multi-mode treatment laser radiation from the additional treatment laser source of the medical treatment device. Similarly, in embodiments, where the medical treatment device further comprises an additional sensor module, e.g. a Raman sensor, the optical combiner module 2700 further has an optical interface 2707 for receipt of single-mode or few-mode sensor laser radiation from the additional sensor module of the medical treatment device and an optical interface 2708 for feeding multi-mode response radiation, which has been reflected by the tissue to be treated in response to being illuminated by the sensor laser radiation from the additional sensor module, back to the additional sensor module of the medical treatment device. It will be appreciated that alternative embodiments of the combiner module 2700 may have fewer, additional and/or alternative optical interfaces to fewer, additional and/or alternative optical modules of the medical treatment apparatus.

The optical combiner module 2700 is configured to combine the optical paths fed through the core of the at least double-clad optical fiber with one another. The optical combiner module 2700 is further configured to combine the optical paths fed through the inner cladding of the at least double-clad optical fiber.

To this end, the optical combiner module 2700 comprises suitable fused fiber combiners for coupling the signal paths fed through the single-mode core of the at least double-clad optical fiber 1200. In particular, the optical combiner module 2700 comprises a first fused fiber combiner 2710, e.g. a single-mode power splitter, which is connected to optical interfaces 2710 and 2702 via respective single-mode optical fibers 2741 and 2742, respectively. The optical combiner module 2700 further comprises a second fused fiber combiner 2720, e.g. configured for wavelength multiplexing, which is connected to the first fused fiber combiner 2710 via single-mode fiber 2743 and connected to optical interface 2703 via single-mode optical fiber 2744. The second fused fiber combiner 2720 may be operable to perform wavelength multiplexing between single-mode radiation at the wavelength of the interferometric distance sensor and few-mode radiation at the wavelengths of the spectroscopic sensor module. In embodiments with an additional sensor module, the optical combiner module 2700 may further comprise a third fused fiber combiner 2730, e.g. configured for wavelength multiplexing, which is connected to the second fused fiber combiner 2720 via single-mode fiber 2745 and connected to optical interface 2707 via single-mode optical fiber 2746. The combined single-mode and few-mode radiation is output by the optical combiner module via single mode fiber 2747. The single-mode fibers 2741-2747 are single-mode at least at the wavelength of the sensor laser radiation of the interferometric sensor module. The single-mode fibers 2741-2747 may have cores having a diameter of less than 15 $\mu$m, such as less than 10 $\mu$m. The single-mode fibers 2741-2747 may have a V number of less than 10 at the respective wavelengths being combined.

The optical combiner module 2700 further comprises suitable free-space components 2750, e.g. including lenses and dichroic filters, to perform multi-mode power and wavelength splitting/combination of the radiation fed through the inner cladding of the at least double-clad optical fiber. To this end, the free-space components are optically coupled via respective multi-mode fibers 2781-2784 to optical interfaces 2704, 2705, 2706 and 2708, respectively. The combined multi-mode radiation is output by the optical combiner module via multi-mode fiber 2785. The multi-mode fibers 2781-2785 may have cores of diameters between 30 $\mu$m and 500 $\mu$m, such as between 50 $\mu$m and 400 $\mu$m, such as between 100 $\mu$m and 200 $\mu$m. The multi-mode fibers 2781-2785 may have a V number of 20 or more.

Accordingly, the optical combiner module 2700 comprises two optical interfaces to be coupled to the disposable fiber-optic device: a single-mode or few-mode interface 2810 (e.g. characterizable by a V number smaller than 10) and a multi-mode interface 2820 (e.g. characterizable by a V number larger than 20), respectively.

Generally, in various embodiments, the medical treatment apparatus comprises an optical combiner module comprising one or more fused fiber couplers configured to multiplex sensor laser radiation from the interferometric distance sensor with sensor laser radiation from the spectroscopic sensor in to an optical fiber that is single-mode at least at a wavelength of the sensor laser radiation of the interferometric distance sensor. In particular, the wavelength(s) of the sensor laser radiation of the interferometric distance sensor may be different from the wavelength(s) of the spectroscopic sensor. The fiber couplers may be fused 3 dB couplers.

In various embodiments, the medical treatment apparatus comprises an optical combiner module configured to multiplex multi-mode treatment laser radiation and multi-mode sensor radiation into a multi-mode optical fiber. To this end, the optical combiner module may include free space components including one or more lenses and one or more dichroic mirrors. The multi-mode sensor radiation may include multi-mode response radiation, which has been reflected by the tissue to be treated in response to being illuminated by sensor laser radiation emitted by the spectroscopic sensor module.

Various embodiments of operating a medical treatment apparatus will now be described. Generally, in various embodiments, the medical treatment apparatus comprises a control unit that is configured, responsive to a received user input, to:

> a) activate a first optical sensor module, in particular an interferometric distance sensor, to sense the distance between the distal end of the at least double-clad optical fiber and the tissue to be treated;
> b) activate a second optical sensor module, in particular a spectroscopic sensor, to detect one or more spectroscopic properties of the tissue to be treated;
> c) control the treatment laser source at least in part in dependence of the sensed distance and/or of the detected properties.

For example, controlling the treatment laser source at least in part in dependence of the sensed distance and/or of the detected properties may comprise that the control unit selects a laser power and/or another operational parameter of the treatment laser radiation at least in part in dependence of the sensed distance and/or of the detected properties; and controls the treatment laser source to emit treatment laser radiation at the selected laser power and/or the selected other operational parameter. Examples of other operational parameters may include a duty cycle, pulse with, etc. of a pulse train of laser pulses, a duration of emission of the treatment laser radiation, and/or the like. The control unit may be configured to control the treatment laser source at least in part in dependence of the sensed distance and/or of the detected properties so as to control the laser energy deposited at the treatment site.

Preferably, the control unit is configured to repeat acts a) through c) while said user input is received or until a termination input is received.

In this respect, the control unit may be configured to automatically control the treatment laser source at least in part in dependence of the sensed distance and/or of the detected properties, or the control unit may be configured to perform said control in a operator-assisted manner. For example, the control unit may be configured to:

- calculate or otherwise select a laser power and/or another operational parameter of the treatment laser radiation at least in part in dependence of the sensed distance and/or of the detected properties;
- display otherwise communicate the calculated or otherwise selected laser power and/or other operational parameter to the operator operating the apparatus and request approval/manual activation of the displayed/communicated values and/or to allow the operator to change and activate the values manually,
- control the treatment laser source at least in part in dependence of the operator-approved or operator-changed laser power and/or other operational parameter.

In various embodiments described herein, time-division multiplexing the treatment laser radiation and optical measurements based on sensor laser radiation using the same at least-double clad optical fiber allows the fiber to be very thin while avoiding detrimental interference between the sensing system and the treatment laser, e.g. caused by retroreflected treatment laser radiation impinging on the detector of the sensor module. In particular, in various embodiments, the medical treatment apparatus is configured to time-division multiplex an interferometric distance sensor signal, a spectroscopic sensor signal and a treatment laser radiation for use in surgery, in particular minimally invasive surgery, e.g. surgery of cancer in the urinary bladder.

The user input and/or the termination signal may be received via a foot pedal or other suitable user activated input device.

In order to efficiently perform time-division multiplexing, the medical treatment device may be operable to switch the various laser sources sufficiently rapidly, e.g. within 1 ms or faster. To this end, when one or more of the laser sources, e.g. the treatment laser source, is a diode laser, rapid switching may be performed by directly switching of the drive current. When the interferometric distance sensor includes an SLED or a swept source, or when the spectroscopic sensor includes a supercontinuum laser, these may need to be switched in another manner in order to obtain rapid switching, as such lasers may need time to stabilize when switched on. Accordingly, the switching may e.g. be performed by means of an acousto-optic tunable filter (AOTF), a MEMS based device or in another suitable manner. Such an external switch can give rise to reflections. Therefore, when used to switch the sensor laser radiation of the interferometric distance sensor, it may preferably be arranged between the sensor laser source and the coupler/circulator for the receiver. In the spectroscopy engine, the switch may be located on the laser output.

In some embodiments, the control unit is configured to receive information about the disposable fiber-optic device connected to the medical treatment device; and to select the laser power at least in part in dependence of a user-selected laser power, the sensed distance and the received information about the disposable fiber-optic device connected to the medical treatment device.

In some embodiments, the control unit is configured to output a camera control signal operable to control operation of a camera of the endoscope, wherein the control unit is configured to synchronize operation of the camera with the operation of the treatment laser source such that image data is recorded by the camera only when the medical treatment apparatus does not emit treatment laser radiation or only emits treatment laser radiation at a substantially reduced intensity, in particular reduced such that the reduced treatment radiation does not unduly affect the camera images. To this end, the treatment laser radiation may be reduced to 5% or less of the intensity of the treatment laser radiation during the preceding treatment cycle, e.g. to 1% or less.

In some embodiments, the control unit is configured to control the laser power of the treatment laser radiation responsive to a detected type of material. To this end, the first optical sensor module may further be configured to detect and distinguish a plurality of different types of material located in front of the distal end of the at least double-clad optical fiber.

FIG. 12 diagrammatically illustrates operation of an example of a fiber-optic medical treatment apparatus in accordance with various embodiments disclosed herein, e.g. of the medical treatment apparatus described in connection with one or more of FIGs. 2 -11, in particular an apparatus for treatment of the urinary bladder. The process is controlled by the control unit of the medical treatment apparatus.

Initially, e.g. responsive to activation of the medical treatment device by a user input, the medical treatment device may be operated in a safety mode. The activation by the user input may e.g. involve the user pushing a button or selecting a mode of operation. In the safety mode, the interferometric distance sensor module of the medical treatment device may be activated and feed sensor laser radiation 4100 through the at least double-clad optical fiber of the disposable fiber-optic device connected to the medical treatment device. The interferometric distance sensor module further receives reflected radiation that is received by the distal end of the at least double-clad optical fiber in response to the emitted sensor laser radiation. The medical treatment device is configured to analyze the received reflected radiation so as to detect whether the distal end is immersed in a liquid, in particular water, and/or whether the distal end is positioned close to a surface other than biological tissue. An example of this detection is described below with reference to FIG. 13. If the distal end is close to a surface other than biological tissue or if the distal end is not immersed in a liquid, in particular water, the control unit may prevent the medical treatment device from emitting treatment laser radiation. Detection of a surface other than biological tissue may indicate that the distal end of the at least double-clad fiber has not been inserted into the endoscope yet or at least has not complete passed through the working channel of the endoscope, or that the endoscope is bent such that the distal end is actually directed towards the outside of the endoscope. Accordingly, preventing the medical treatment device from emitting the treatment laser radiation in this situation prevents unintended damage of the endoscope or other surfaces by the medical treatment laser radiation.

Failure to detect a liquid, in particular water, may indicate that the distal end of the at least double-clad fiber has not yet been positioned close to the treatment site, in particular not yet been inserted into the urinary bladder. Preventing the medical treatment device from emitting the treatment laser radiation in this situation prevents unintended damage of tissue other than the intended tissue.

Preventing emission of the treatment laser radiation may include preventing the treatment laser source from being powered on and/or preventing a suitable shutter/optical switch from being opened or preventing emission of the treatment laser radiation in another suitable manner.

When, in particular only when, the medical treatment device detects biological tissue in a proximity of the distal end of the at least double-clad optical fiber and water between the distal end and the detected biological tissue, the control unit allows the medical treatment device to exit the safety mode and enter treatment mode.

When operated in treatment mode, the operator can initiate emission of the treatment laser radiation by activating a suitable user input device, e.g. a foot pedal or the like. Upon activation of the user input device, the control unit receives an activation signal 4200, e.g. as long as the foot pedal maintains activated. In some embodiments each activation of the foot pedal may cause emission of a single burst of treatment laser radiation (or another predetermined number of bursts), e.g. corresponding to a predetermined - e.g. user selected - amount of laser energy to be delivered to the tissue to be treated (e.g. a predetermined amount of laser energy per unit area).

Accordingly, in some embodiments, responsive to the user command, the apparatus may emit a single burst or a series of e.g. 2, 3, 4 or 5 bursts of laser radiation, in particular at high laser power, e.g. between 10 W and 200 W. The user command may e.g. involve the user operating a foot pedal. For example, the apparatus may be configured to emit a predetermined amount of laser energy as a single burst or as a series of few bursts of laser radiation. The predetermined amount of energy may be user selected. In some embodiments, the predetermined amount of energy may at least in part be determined responsive to a measured distance between the distal end and the tissue to be treated, e.g. so as to deliver a predetermined amount of energy per unit area of the tissue being illuminated by the treatment laser radiation. The amount of energy may be controlled by adjusting the output power and/or the burst duration and/or - in case of the emission of pulsed radiation, a duty cycle of the pulsed laser radiation.

Responsive to the activation signal, the control unit repeatedly performs a treatment cycle, e.g. as long as the activation signal 4200 is received or until a termination signal is received, or for a predetermined amount of time, e.g. so as to emit a single burst of laser radiation.

During each treatment cycle, the control unit initially causes the interferometric distance sensor to emit sensor laser radiation 4110 and to sense the distance between the distal end of the at least double-clad optical fiber and the tissue to be treated. The distance sensor may perform one or more distance measurements during a suitable measurement period, e.g. 10 ms or less. For example, the control unit may compute an average over a plurality of distance measurements. If the interferometric distance sensor detects that the surface in front of the distal end is a surface other than biological tissue or if the distal end is not immersed in a liquid, in particular water, the control unit causes the medical treatment device to exit the treatment mode and return to the safety mode.

Otherwise, upon completion of the distance measurement, the control unit deactivates the sensor laser radiation from the interferometric distance sensor and activates the spectroscopic sensor to emit the spectroscopy sensor laser radiation 4310. The spectroscopic sensor receives light reflected by the tissue to be treated in response to being illuminated with the spectroscopic laser radiation and determines a state/progress of the treatment, e.g. based on an absolute or relative reflected intensity or power in one or more wavelength bands, e.g. at emission peaks of hemoglobin or another suitable molecular marker indicative of the progress of the laser treatment. The spectroscopic measurement may last a suitable measurement period of time, e.g. 10 ms or less. The control unit may then deactivate the sensor radiation from the spectroscopic distance sensor.

Based at least in part on the determined distance and/or on the determined progress, in step 4410 the control unit determines the power and/or duty cycle or other suitable parameter of the treatment laser radiation to be emitted. For example, the control unit may select a smaller power and/or duty cycle if the measured distance is small and a larger power and/or duty cycle if the measured distance is large. The control unit may even prevent emission of the treatment laser radiation if the detected distance is smaller than a safety threshold.

In some embodiments, the selection of the power (or other suitable parameter) of the treatment laser radiation may further be based on a user-selected baseline power (or other suitable parameter). For example, the control unit may automatically adjust the baseline power responsive to the detected distance, e.g. attenuate the laser power relative to the baseline power responsive to a reduced distance.

Alternatively or additionally, the control unit may select the power, duty cycle and/or other suitable parameter of the treatment laser radiation responsive to the detected status/progress of the treatment, e.g. attenuate the laser power and/or duty cycle responsive to an increased progress.

The control unit may further receive information about characteristics of the disposable fiber-optic device currently connected to the medical treatment device, e.g. by reading out an ID tag, e.g. an RFID tag, a OR code or other machine-readable identifier. The control unit may thus select the laser power based on the sensor data from the distance sensor (and, optionally, the sensor data from the spectroscopic sensor), from the pre-set baseline power at the time of device activation and from the received information about the characteristics of the disposable fiber-optic device. The information of the characteristics of the disposable fiber-optic device may e.g. include information about the fiber diameter, numerical aperture and/or the like.

The control unit then controls the treatment laser to emit the treatment laser radiation 4510 at the selected power. To this end, the control unit may control the treatment laser source to output treatment laser radiation at a suitable output power, e.g. a selected output power in the interval between 10 W and 2200 W or in another suitable interval. In some embodiments the treatment laser radiation may be emitted as a pulse train of treatment laser pulses. The pulses may have a pulse duration of between 1 ms and 10 ms or another suitable pulse duration. The control unit may control the treatment laser to emit the treatment laser radiation for a treatment period which may be predetermined, e.g. as a pre-configured duration or user-selected, or the treatment period may at least partly be automatically selected based on the sensed distance and/or detected progress. In some embodiments, the treatment period may correspond to between 5 and 100 laser pulses, e.g. between 10 and 20 pulses. Upon completion of the treatment period, the control unit deactivates emission of the treatment laser radiation.

Upon completion of the treatment cycle, the control unit again controls the interferometric distance sensor to perform a distance measurement by emitting corresponding sensor laser radiation 4120, followed by a spectroscopic measurement 4320, as described above.

Based at least in part on the determined distance and/or on the determined progress, in step 4420 the control unit determines the power, duty cycle and/or other suitable parameter of the treatment laser radiation to be emitted during the subsequent treatment period. In some embodiments, the determination of the laser power (or other parameter) is not only based on the most recent measurements but further on measurements made during one or more previous treatment cycles. To this end, the control unit may include a memory for storing the results of one or more previous distance measurements and/or status/progress measurements. The results of one or more previous distance measurements may e.g. be used to determine changes in distance due to relative movement of the distal end of the at least double-clad optical fiber and the tissue to be treated. The control unit may thus base the selection of the laser power or other parameter not only on the currently measured distance but also on an observed change of distance, e.g. on rate of change, e.g. whether the distance increases or decreases and, optionally, at what speed. Similarly, the control unit may base the selection of the laser power or other parameter on an observed rate of change of the treatment status.

In some embodiments, the control unit further bases the selected laser power or other parameter on patient feedback received via a suitable user device, e.g. a handheld user device, operated by the patient during the course of the treatment. For example, the patient may activate the user device when the patient experiences pain or discomfort during the treatment. The user device may communicate a corresponding patient feedback signal 4700 to the control unit, which may then adjust the laser power or other parameter for the subsequent treatment cycle responsive to the received feedback.

The control unit then controls the treatment laser to emit treatment laser radiation 4520 at the selected power and/or other parameter for another treatment period.

The control unit may repeatedly perform treatment cycles as long as the activation signal 4200 is activated or until a termination signal is received or for a predetermined treatment duration, e.g. so as to emit a predetermined burst of laser radiation. As mentioned above, the control unit may also interrupt the treatment responsive to detected unsafe conditions, e.g. that the fiber is directed at a non-tissue surface, that the fiber is no longer immersed in water and/or too close to the tissue to be treated, or when the spectroscopic measurement indicates that the treatment of the tissue has been completed.

In some embodiments, the endoscope used together with an embodiment of the medical treatment apparatus disclosed herein may include a camera, and the endoscope may be communicatively coupled to the medical treatment device so as to allow the medical treatment device to control at least some functions of the endoscope. In particular, the control unit of the medical treatment device may be operable to control operation of the camera of the endoscope. To this end, the control unit may be operable to output a camera control signal 4600 configured to control the camera to only capture image data when the treatment laser radiation is off, e.g. only during the measurement periods and/or during the intervals between laser pulses. Accordingly, possible interference of the treatment laser radiation with the camera image are avoided.

It will be appreciated that several modifications may be made to the process for operating the medical treatment apparatus. For example, if the medical treatment apparatus includes further sensors, measurement periods of these additional sensors may also be included in one or more of the treatment cycles, e.g. in a time-division multiplexed manner as was described above in respect of the distance measurements and spectroscopic measurements. In some embodiments, some or all of the measurements, e.g. the spectroscopic measurement, may not necessarily be performed during each treatment cycle but may e.g. only be performed during a subset of treatment cycles.

FIGs. 13A-B illustrate examples of distance sensing signals obtained by a sensor module of a fiber-optic medical treatment apparatus in accordance with various embodiments disclosed herein.

In particular, FIG. 13A shows an example of a measured interferometric distance signal, where the interferometric distance sensor is configured to emit sensor laser radiation within a predetermined wavelength range and to record the power of the corresponding reflected radiation. FIGs. 13B and 13C show examples of a depth scan calculated by a Fourier-transform from acquired spectra of the interferometric distance sensor. In particular, FIGs. 13B and 13C show respective plots of the calculated Fourier power as a function of the distance from the distal end of the at least double-clad optical fiber. In the example of FIG. 13B biological tissue was positioned about 1.25 mm in front of the distal end of the fiber, as indicated by dashed line 5100. In the example of FIG. 13C, a flat, reflective surface was positioned about 1.8 mm in front of the distal end of the fiber, as indicated by dashed line 5200.

From a comparison of the depth scans of FIGs. 13B and 13C it is apparent that biological tissue and an artificial surface such as the interior or exterior surface of an endoscope can easily be distinguished based on the interferometric measurement signals, e.g. by peak detection and peak analysis techniques known as such in the art of signal processing. Similarly, the depths scans can be used to detect the presence or absence of water in the space between the distal end and a surface.

Various aspects have been described with reference to various embodiments. Modifications and alterations will occur to others upon reading the present disclosure.

It is intended that the invention be construed as including all such modifications and alterations, including insofar as they come within the scope of the appended claims and the equivalents thereof.

Some embodiments of aspects disclosed herein may be summarized as follows:

Embodiment 1: A fiber-optic medical treatment apparatus comprising a medical treatment device and a disposable fiber-optic device, wherein the disposable fiber-optic device comprises an at least double-clad optical fiber having a proximal end and a distal end, the distal end being configured to be advanced through a working channel of an endoscope, wherein the at least double-clad optical fiber has at least one core and two or more claddings; wherein the medical treatment device comprises:

- a control unit configured to control operation of the medical treatment device;
- a treatment laser source configured to output treatment laser radiation for treatment of a medical condition; wherein the medical treatment apparatus is configured to output the treatment laser radiation through at least one of the two or more claddings of the at least doubled-clad optical fiber towards tissue to be treated;

- a sensor module configured to output and receive sensor laser radiation through the at least doubled-clad optical fiber and to optically measure one or more parameters of the treatment based on the received sensor laser radiation; wherein the at least one core of the at least double-clad fiber has a V number of less than 10 at a wavelength of the sensor laser radiation, wherein the medical treatment apparatus is configured to output the sensor laser radiation through the at least one core of the at least doubled-clad optical fiber, and wherein the medical treatment apparatus is configured to time-division multiplex the output of the treatment laser radiation and at least the optical measurement of the one or more parameters.

Embodiment 2: A fiber-optic medical treatment apparatus according to embodiment 1, wherein the disposable fiber-optic device further comprises:

- a connector module for optically connecting the proximal end of the at least double-clad optical fiber to the medical treatment device, wherein the connector module comprises:
- a first optical fiber,
- a second optical fiber,
- a first optical connector for detachably and optically connecting the first optical fiber to the medical treatment device,
- a second optical connector for detachably and optically connecting the second optical fiber to the medical treatment device, and
- an optical combiner module configured to couple radiation between the first optical fiber and the at least one core of the at least double-clad optical fiber and to couple radiation between the second optical fiber and the at least one cladding of the at least double-clad optical fiber.

Embodiment 3: A fiber-optic medical treatment apparatus according to any one of the preceding embodiments, wherein the sensor module comprises a first optical sensor module configured to perform interferometric distance sensing of a distance between the distal end of the at least double-clad optical fiber and the tissue to be treated. Embodiment 4: A fiber-optic medical treatment apparatus according to embodiment 3; wherein the control unit is configured to control a laser power and/or other operational parameter of the treatment laser radiation responsive to the sensed distance.

Embodiment 5: A fiber-optic medical treatment apparatus according to embodiment 3 or 4; wherein the first optical sensor module is further configured to detect and distinguish a plurality of different types of material located in front of the distal end of the at least

double-clad optical fiber, and wherein the control unit is configured to control a laser power and/or other operational parameter of the treatment laser radiation responsive to the detected type of material.

Embodiment 6: A fiber-optic medical treatment apparatus according to any one of the preceding embodiments, wherein the sensor module comprises a second optical sensor module configured for spectroscopic analysis of the tissue to be treated and to compute an indicator of a progress of the treatment.

Embodiment 7: A fiber-optic medical treatment apparatus according to embodiment 6 when dependent on any of embodiments 3 through 5, wherein the control unit is configured, responsive to a received user input, to:

    a) activate the first optical sensor module to sense the distance between the distal end of the at least double-clad optical fiber and the tissue to be treated;
    b) activate the second optical sensor module to detect one or more spectroscopic properties of the tissue to be treated;
    c) control the treatment laser source at least in part in dependence of the sensed distance and/or of the detected properties.

Embodiment 8: A fiber-optic medical treatment apparatus according to embodiment 7; wherein the control unit is configured to repeat acts a) through c) while said user input is received or until a termination input is received.

Embodiment 9: A fiber-optic medical treatment apparatus according to embodiment 7 or 8; wherein the control unit is configured to receive information about the disposable fiber-optic device connected to the medical treatment device; and to control the treatment laser source at least in part in dependence of a user-selected operational parameter of the laser source and/or the received information.

Embodiment 10: A fiber-optic medical treatment apparatus according to any one of embodiments 7 through 9, wherein the control unit is configured to output a camera control signal operable to control operation of a camera of the endoscope, wherein the control unit is configured to synchronize operation of the camera with the operation of the treatment laser source such that image data is recorded by the camera only when the medical treatment apparatus does not emit treatment laser radiation or only emits treatment laser radiation at a substantially reduced intensity.

Embodiment 11: A fiber-optic medical treatment apparatus according to any one of the preceding embodiments, wherein the medical treatment device comprises an audible and/or visible power indicator configured to output an audible and/or visual indication indicative of a current laser power of the treatment laser radiation.

Embodiment 12: A fiber-optic medical treatment apparatus according to any one of the preceding embodiments, further comprising a handheld communication device communicatively coupled to the medical treatment device, the handheld communication device comprising a user input device configured to receive user input from a patient during treatment with the medical treatment apparatus, the user input being indicative of discomfort experienced by the patient during treatment; and wherein the control unit is configured to select a laser power and/or other operational parameter of the treatment laser radiation at least in part responsive to the received user input.

Embodiment 13: A fiber-optic medical treatment apparatus according to any one of the preceding embodiments, wherein the at least one core is a few-mode core or a single-mode core at least at a wavelength of the sensor laser radiation.

Embodiment 14: A fiber-optic medical treatment apparatus according to any one of the preceding embodiments, wherein the at least two claddings include a multi-mode inner cladding surrounding the at least one core, the inner cladding being surrounded by at least one outer cladding.

Embodiment 15: A fiber-optic medical treatment apparatus according to any one of the preceding embodiments wherein the treatment laser radiation is multi-mode radiation and the sensor laser radiation includes single-mode and/or few-mode laser radiation.

**Claims**

1. A system for treatment of the urinary tract, the system comprising a medical treatment apparatus and a flexible endoscope, the endoscope being adapted for insertion into the urinary tract of a patient and having a working channel, wherein the medical treatment apparatus comprises:

    - a treatment laser source and an at least double-clad optical fiber, wherein the at least double-clad optical fiber has a proximal end and a distal end, the distal end being configured to be advanced through the working channel of the endoscope, wherein the treatment laser source is configured for emitting treatment laser radiation through the at least double-clad optical fiber towards tissue to be treated,
    - a first optical sensor module configured to perform optical distance measurements using said at least double-clad optical fiber,
    - a control unit configured, responsive to a received user input, to:

        a) activate the first optical sensor module to perform the optical distance measurement;
        b) control the treatment laser source at least in part in dependence of the sensed distance.

2. The system according to claim 1, wherein the medical treatment apparatus is configured to time-division multiplex output of the treatment laser radiation and at least the optical distance measurement.

3. The system according to any one of the preceding claims, wherein the medical treatment apparatus further comprises a second optical sensor module operable to sense one or more spectroscopic properties of the tissue to be treated, wherein the first and second optical sensor modules are configured to perform optical measurements using said at least double-clad optical fiber.

4. The system according to claim 3, wherein the control unit is configured, responsive to a received user input, to:

        a) activate the first optical sensor module to perform the optical distance measurement;
        b) activate the second optical sensor module to detect the one or more spectroscopic properties of the tissue to be treated;
        c) control the treatment laser source at least in part in dependence of the sensed distance and/or of the detected properties.

5. The system according to any one of claims 3 through 4, wherein the medical treatment apparatus is configured to time-division multiplex output of the treatment laser radiation and at least the optical distance measurement and the optical measurement of the one or more spectroscopic properties.

6. A fiber-optic medical treatment apparatus according to any one of claims 3 through 5, wherein the second optical sensor module is configured for spectroscopic analysis of the tissue to be treated and to compute an indicator of a progress of the treatment.

7. The system according to any one of the preceding claims, wherein the at least double-clad optical fiber has a diameter between 0.1 mm and 1 mm, in particular between 0.1 mm and 0.4 mm.

8. The system according to any one of the preceding claims, wherein the at least double-clad optical fiber comprises a multi-mode cladding for conveying the treatment laser radiation from the treatment laser source, the multi-mode cladding having a numerical aperture of between 0.17 and 0.50, in particular between 0.22 and 0.45.

9. The system according to any one of the preceding claims, wherein the control unit is configured to automatically control the treatment laser source at least in part in dependence of the sensed distance, or to perform said control in an operator-assisted manner.

10. The system according to claim 9, wherein the control unit is configured to:

    - calculate or otherwise select a laser power and/or another operational parameter of the treatment laser radiation at least in part in dependence of the sensed distance;
    - display or otherwise communicate the calculated or otherwise selected laser power and/or other operational parameter to the operator operating the medical treatment apparatus and request approval/manual activation of the displayed/communicated values and/or to allow the operator to change and activate the values manually,
    - control the treatment laser source at least in part in dependence of the operator-approved or operator-changed laser power and/or other operational parameter.

11. The system according to any one of the preceding claims, wherein the medical treatment apparatus comprises a medical treatment device and a disposable fiber-optic device, wherein the disposable fiber-optic device comprises:

    - the at least double-clad optical fiber, and
    - a connector module for optically connecting the proximal end of the at least double-clad optical fiber to the medical treatment device.

12. The system according to claim 11, wherein the connector module comprises:

    - a first optical fiber,
    - a second optical fiber,
    - a first optical connector for detachably and optically connecting the first optical fiber to the medical treatment device,
    - a second optical connector for detachably and optically connecting the second optical fiber to the medical treatment device, and
- an optical combiner module configured to couple radiation between the first optical fiber and the at least one core of the at least double-clad optical fiber and to couple radiation between the second optical fiber and the at least one cladding of the at least double-clad optical fiber.

13. The system according to any one of the preceding claims, wherein the first optical sensor module is configured to perform interferometric distance sensing.

14. The system according to claim 13, wherein the first optical sensor module is further configured to detect and distinguish a plurality of different types of material located in front of the distal end of the at least double-clad optical fiber, and wherein the control unit is configured to control a laser power and/or other operational parameter of the treatment laser radiation responsive to the detected type of material.

15. The system according to any one of the preceding claims wherein the treatment laser radiation is multi-mode radiation and the sensor laser radiation includes single-mode and/or few-mode laser radiation.

*FIG. 1*

*FIG. 2*

*FIG. 3*

1132    1134

1110

1135
1136

1200

1120

1137    1130

1138

*FIG. 4*

1134

1110

1135
1136

1132    1200

1220

1120

1137    1130

1138

*FIG. 5*

1211    1200

1212

1213

1212    1214

*FIG. 6*

1224    1225    1201

1223

1222

1221    1200

1220

*FIG. 7*

1200

1213
1211

1214

*FIG. 8A*

1213    1200
1211

1214

*FIG. 8B*

1211    1200
1213

1212
1214

*FIG. 8C*

1211    1200
1213

1214
1212    1215

*FIG. 8D*

1216
1213    1211
1212

1200

1214    1215

*FIG. 8E*

1200

1213    1211
1217

1218
1214

*FIG. 8F*

1200    1201

1213

*FIG. 9A*

1200    1201

1213

*FIG. 9B*

1201

1213

*FIG. 9C*    1213

1200

a

FIG. 10

EP 4 714 384 A2

FIG. 11

FIG. 12

*FIG. 13A*

*FIG. 13B*

*FIG. 13C*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021026164 A